# EUROPEAN PATENT SPECIFICATION

(11) **EP 3 064 178 B1**
(45) Date of publication and mention of the grant of the patent: **29.11.2017**
(21) Application number: 14859258.7
(22) Date of filing: 01.10.2014
(51) Int. Cl.: A61F 13/15, A61F 13/472, A61F 13/49

(54) **MANUFACTURING DEVICE AND MANUFACTURING METHOD OF COMPOSITE BODY OF CONTINUOUS SHEET ASSOCIATED WITH ABSORBENT ARTICLE**
HERSTELLUNGSVORRICHTUNG UND HERSTELLUNGSVERFAHREN FÜR FOLIENBAHNVERBUND FÜR EINEN SAUGFÄHIGEN ARTIKEL
DISPOSITIF DE FABRICATION ET PROCÉDÉ DE FABRICATION DE COMPOSITE EN FEUILLE CONTINUE POUR ARTICLE ABSORBANT

(30) Priority: 30.10.2013 JP 2013225326
(43) Date of publication of application: 07.09.2016
(73) Proprietor: Unicharm Corporation, Ehime 799-0111 (JP)
(72) Inventor: SIOMI, Akihisa, Kanonji-shi Kagawa 769-1602 (JP); MYOJO, Ryota, Kanonji-shi Kagawa 769-1602 (JP); AKANO, Yukihisa, Kanonji-shi Kagawa 769-1602 (JP); YOKOYAMA, Takumi, Kanonji-shi Kagawa 769-1602 (JP)
(74) Representative: Dolleymores
(86) International application number: PCT/JP2014/076300
(87) International publication number: WO 2015/064286

(56) References cited:
- EP-A1- 2 554 143
- JP-A- 2003 508 243
- JP-A- 2010 063 716
- JP-A- 2010 142 415
- JP-A- 2010 142 415
- JP-A- 2011 189 070
- JP-A- 2012 024 242
- US-A1- 2013 213 570

## Description

### [Technical Field]

The present invention relates to a manufacturing device and a manufacturing method of a composite body of a continuous sheet associated with an absorbent article such as a disposable diaper.

### [Background Art]

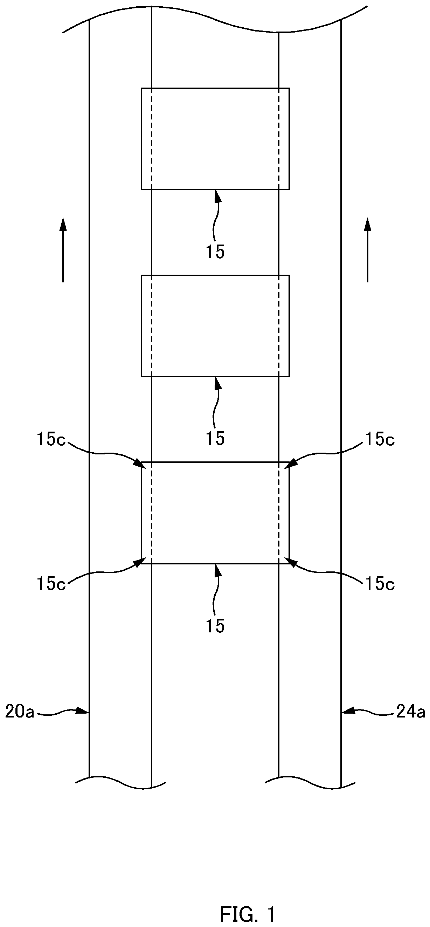

In a conventional manufacturing line of an absorbent article such as a disposable diaper, a process is performed in which a sheet-like member 15 including four corners 15c, 15c, 15c, 15c is extended across and bonded to a pair of continuous sheets 20a, 24a traveling along a continuous direction, as illustrated in FIG. 1.

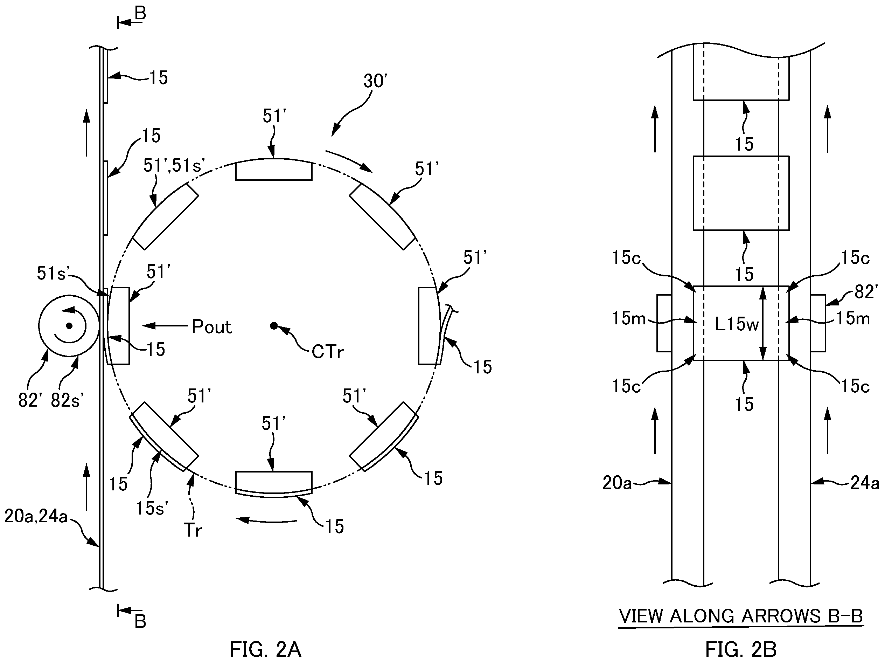

Such a process is performed, for example, using a rotating dram device 30'. FIG. 2A is a schematic side view of the rotating dram device 30', and FIG. 2B is a view along arrows B-B in FIG. 2A.

As illustrate in FIG. 2A, the rotating dram device 30' includes a plurality of holding pads 51', 51' that moves along a circulating trajectory Tr having a perfect circle shape about a predetermined rotational axis CTr. Each of the holding pads 51', 51' includes a holding surface 51s' that holds one side of the aforementioned sheet-like member 15. The holding surface 51s' is formed into an ark-like curved surface, and when the holding pad 51' passes a predetermined position Pout of the circulating trajectory Tr, the ark shape of the above-mentioned ark-like curved surface is in a state of being along the circulating trajectory Tr.

Further, at the predetermined position Pout, the pair of continuous sheets 20a, 24a travels alongside in a direction along the aforementioned rotational axis Ctr through a travel path so as to substantially contact the circulating trajectory Tr (FIG. 2B). Further, a roller member 82' is arranged at the predetermined position Pout. When the holding pad 51' passes the predetermined position Pout, the pair of continuous sheets 20a, 24a is pressed against and bonded to the sheet-like member 15 by an outer peripheral face 82s' of the roller member 82', and thus the sheet-like member 15 is fixed in a state of being extended across the pair of continuous sheets 20a, 24a. Then, the sheet-like member 15 is delivered as it is to the pair of continuous sheets 20a, 24a from the holding surface 51s'.

### [Citation List]

### [Patent Literature]

PTL 1: Japanese Translation of PCT International Application Publication No. 2005-075163

### [Summary of the Invention]

### [Technical Problem]

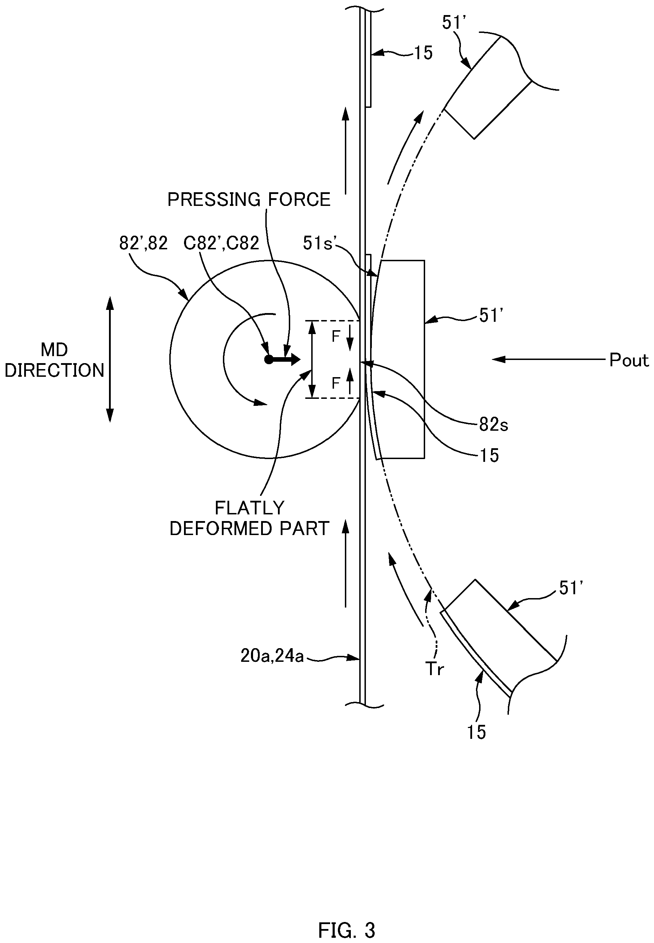

Here, as illustrated in FIG. 3, the aforementioned pressing causes the outer peripheral face 82s' of the roller member 82' to flatly deform, and a force F that compresses in a circumferential direction of the roller member 82' may act on on the sheet-like member 15 due to the flattened deformation. This may cause small creases on the sheet-like member 15. Then, such creases may deteriorate the appearance of the diaper and may become a cause of an uncomfortable feeling and a leakage when worn.

On the other hand, in view of bonding and fixing property of the sheet-like member 15 with respect to the pair of continuous sheets 20a, 24a, it is not necessary for the sheet-like member 15 to be bonded to the pair of continuous sheets 20a, 24a with a high bonding strength over the entire length L15w (Fig. 2B) of the sheet-like member 15 with respect to a direction along the circulating trajectory Tr. If the sheet-like member 15 is bonded with a high bonding strength at least at four corners 15c, 15c, 15c, 15c, it is considered that the sheet-like member 15 can be substantially firmly fixed to the pair of continuous sheets 20a, 24a even if a part 15m (FIG. 2B) between two corners 15c, 15c adjacent with each other in a direction along the circulating trajectory Tr is bonded with a lower bonding strength than that of the four corners 15c, 15c. Then, for this reason, it is considered that there is no problem even if the pressing force of the roller member 82' in the part 15m between the two corners 15c, 15c is lower than that of the four corners 15c, 15c, and thus it may be possible to suppress creases of the sheet-like member 15 due to the reduction of the flattened deformation of the roller member 82' .

The present invention was achieved in light of the above-described problems, and an object thereof is to suppress creases that may occur on the sheet-like member and firmly fix the sheet-like member in a state of extending across a pair of continuous sheets.

### [Solution to Problem]

A principal aspect of the present invention to achieve the above advantage is
a manufacturing device of a composite body of a continuous sheet associated with an absorbent article by extending a sheet-like member having four corners across a pair of continuous sheets that travels in parallel along a continuous direction and bonding the sheet-like member to the pair of continuous sheets, the device including:
a holding member that circulates along a circulating trajectory having a circular shape about a predetermined rotational axis, the holding member holding one side of the sheet-like member on a holding surface that faces outward in a radial direction of the circulating trajectory; and
a roller member arranged according to a predetermined position on the circulating trajectory, an outer peripheral face of the roller member pressing the pair of continuous sheets toward the sheet-like member when the holding surface passes the predetermined position, and thus causing the sheet-like member to be bonded to the pair of continuous sheets and delivered to the pair of continuous sheets from the holding surface,
the holding surface including portions that respectively correspond to the four corners of the sheet-like member, each of the portions being a curve surface-like portion along an arc track that is drawn by the each of the portions when passing the predetermined position,
a track that is drawn when at least a part that is arranged between two adjacent curve surface-like portions in a direction along the circulating trajectory on the holding surface passes the predetermined position being positioned more inwardly in the radial direction of the circulating trajectory than the arc track that is drawn when the two adjacent curve surface-like portions passes the predetermined position,
the holding surface sucking and holding the sheet-like member with a suction force,
a suction force per unit area of the curve surface-like portion on the holding surface being greater than that of a portion other than the curve surface-like portion on the holding surface.

Further,
a manufacturing method of a composite body of a continuous sheet associated with an absorbent article by extending a sheet-like member having four corners across a pair of continuous sheets that travels in parallel along a continuous direction and bonding the sheet-like member to the pair of continuous sheets, the method including:
causing a holding member that holds the sheet-like member on a holding surface to circulate along a circulating trajectory having a circular shape about a predetermined rotational axis, and
when the holding surface passes a predetermined position on the circulating trajectory in a state of facing outward in a radial direction of the circulating trajectory, bonding the sheet-like member to the pair of continuous sheets and delivering the sheet-like member to the pair of continuous sheets from the holding surface by pressing the pair of continuous sheets against the sheet-like member with an outer peripheral face of a roller member arranged according to the predetermined position,
the holding surface including portions that respectively correspond to the four corners of the sheet-like member, each of the portions being a curve surface-like portion along an arc track that is drawn by the each of the portions when passing the predetermined position,
a track that is drawn when at least a part that is arranged between two adjacent curve surface-like portions in a direction along the circulating trajectory on the holding surface passes the predetermined position being positioned more inwardly in the radial direction of the circulating trajectory than the arc track that is drawn when the two adjacent curve surface-like portions passes the predetermined position,
the holding surface sucking and holding the sheet-like member with a suction force,
a suction force per unit area of the curve surface-like portion on the holding surface being greater than that of a portion other than the curve surface-like portion on the holding surface.

Other features of the present invention will be made clear through the present specification with reference to the accompanying drawings.

### [Effects of the Invention]

According to the present invention, a sheet-like member can be firmly fixed in a state of extending across a pair of continuous sheets while creases that may be caused on the sheet-like member are suppressed.

### [BRIEF DESCRIPTION OF THE DRAWINGS]

[Fig. 1] Fig. 1 is an explanatory diagram of a process to be performed in a manufacturing line of an absorbent article such as a disposable diaper 1.
[Fig. 2] Fig. 2A is a schematic side view of a rotating dram device 30' that performs such a process, and FIG. 2B is a view along arrows B-B in FIG. 2A.
[Fig. 3] Fig. 3 is a diagram explaining a reason for causing creases on the sheet-like member 15 due to the pressing of the roller member 82 when the sheet-like member 15 is extended across and delivered to the continuous sheet20a, 24a.
[Fig. 4] Fig. 4 is a schematic plan view of the diaper 1 in a developed state.
[Fig. 5] Fig. 5 is a schematic perspective view of the diaper 1 in a worn state.
[Fig. 6] Fig. 6 is a schematic exploded perspective view of the diaper 1 in a developed state.
[Fig. 7] Fig. 7 is a schematic exploded perspective view of an exterior sheet member 15.
[Fig. 8] FIG. 8A is a schematic side view of a manufacturing device 30 of the present embodiment, FIG. 8B is a view along arrows B-B in FIG. 8A, and FIG. 8C is a view along arrows C-C in FIG. 8A.
[Fig. 9] Fig. 9A is a front view of a holding surface 51s' of the comparative example, FIG. 9B is a view along arrows B-B in FIG. 9A, FIG. 9C is a view along arrows C-C in FIG. 9A.
[Fig. 10] Fig. 10A is a front view of a holding surface 51s of the present embodiment, FIG. 10B is a view along arrows B-B in FIG. 10A, FIG. 10C is a view along arrows C-C in FIG. 10A.
[Fig. 11] Fig. 11 is a schematic perspective view of a holding pad 51 of the present embodiment.
[Fig. 12] Fig. 12 is a diagram showing distribution of magnitude of a friction coefficient on the holding surface 51s.
[Fig. 13] Fig. 13 is a diagram showing an example of the configuration of a holding surface side portion 511 that can be divided into three parts.

### [Description of Embodiments]

At least the following matters will be made clear by the description in the present specification and the accompanying drawings.

A manufacturing device of a composite body of a continuous sheet associated with an absorbent article by extending a sheet-like member having four corners across a pair of continuous sheets that travels in parallel along a continuous direction and bonding the sheet-like member to the pair of continuous sheets, the device including:
a holding member that circulates along a circulating trajectory having a circular shape about a predetermined rotational axis, the holding member holding one side of the sheet-like member on a holding surface that faces outward in a radial direction of the circulating trajectory; and
a roller member arranged according to a predetermined position on the circulating trajectory, an outer peripheral face of the roller member pressing the pair of continuous sheets toward the sheet-like member when the holding surface passes the predetermined position, and thus causing the sheet-like member to be bonded to the pair of continuous sheets and delivered to the pair of continuous sheets from the holding surface,
the holding surface including portions that respectively correspond to the four corners of the sheet-like member, each of the portions being a curve surface-like portion along an arc track that is drawn by the each of the portions when passing the predetermined position,
a track that is drawn when at least a part that is arranged between two adjacent curve surface-like portions in a direction along the circulating trajectory on the holding surface passes the predetermined position being positioned more inwardly in the radial direction of the circulating trajectory than the arc track that is drawn when the two adjacent curve surface-like portions passes the predetermined position.

According to such a manufacturing device of the composite body of the continuous sheet associated with the absorbent article, each of the portions corresponding to the four corners of the sheet-like member on the holding surface is a curve surface-like portion along the above-mentioned arc track. Thus, the holding surface and the roller member can cooperate to certainly press the four corners of the sheet-like member against the pair of continuous sheets. Thereby, the four corners of the sheet-like member can be bonded to the pair of continuous sheets with a high bonding strength, and thus the sheet-like member can be firmly fixed to the pair of continuous sheets in a state of being extended across the pair of continuous sheets.

Further, a track that is drawn by the above-mentioned part arranged between two curve surface-like portions is positioned more inwardly in the radial direction of the circulating trajectory than the arc track that is drawn by the two curve surface-like portions.

Thus, a pressing force per unit area that is imparted from the above-mentioned roller member to the above-mentioned part arranged between the two curve surface-like portions can be made smaller than a pressing force per unit area that is imparted from the roller member to the above-mentioned two curve surface-like portions. Thereby, since the pressing force in the part is lowered, flattened deformation of the outer peripheral face of the roller member can be mitigated and reduced at least in the part, thus suppressing creases of the sheet-like member.

In the manufacturing device of the composite body of the continuous sheet associated with absorbent article,
the holding surface sucks and holds the sheet-like member with a suction force, and
a suction force per unit area of the curve surface-like portion on the holding surface is greater than that of a portion other than the curve surface-like portion on the holding surface.

According to such a manufacturing device of the composite body of the continuous sheet associated with the absorbent article, the holding surface can securely hold the four corners of the sheet-like member with the above-mentioned four curve surface-like portions on the basis of the magnitude relation of the suction force per unit area as described above. Consequently, the sheet-like member can be certainly restrained to be a state of being impossible to be displaced and deformed on the holding surface. This also contributes to suppression of creases of the sheet-like member when being bonded to the pair of continuous sheets.

In the manufacturing device of the composite body of the continuous sheet associated with absorbent article,
it is preferable that a friction coefficient of the curve surface-like portion is greater than that of the part that is arranged between the two curve surface-like portions on the holding surface.

According to such a manufacturing device of the composite body of the continuous sheet associated with the absorbent article, the holding surface can securely hold the four corners of the sheet-like member with the above-mentioned four curve surface-like portions on the basis of the magnitude relation of the friction coefficient as described above. Thus, it is possible to certainly restrain the sheet-like member in a state of being impossible to be displaced and deformed on the holding surface. This also contributes to suppression of creases of the sheet-like member when being bonded to the pair of continuous sheets.

In the manufacturing device of the composite body of the continuous sheet associated with absorbent article,
it is preferable that a treatment for lowering adhesion is applied to the holding surface.

According to such a manufacturing device of the composite body of the continuous sheet associated with the absorbent article, it is possible to prevent adhesion of adhesive to the holding surface and problems accompanying the adhesion in advance. In other words, adhesive is previously applied to the sheet-like member or the continuous sheet to be bonded with each other, and when the pair of continuous sheets is pressed against the sheet-like member of the holding surface by the roller member to be bonded thereto, there is a risk that the adhesive penetrates and oozes out in the thickness direction, and then adheres to the holding surface. In a case where the adhesive adheres thereto, the sheet-like member becomes difficult to be released from the holding surface even after the sheet-like member is bonded to the pair of continuous sheets at the above-mentioned predetermined position. As a result, the sheet-like member is not smoothly delivered to the pair of continuous sheets. However, in this regard, if adhesion of the holding surface is lowered as described above, the adhesion of adhesive to the holding surface can be effectively prevented, thus also preventing the problems described above.

In the manufacturing device of the composite body of the continuous sheet associated with absorbent article,
it is preferable that at the predetermined position, a travel direction of the continuous sheet is along the arc track, and a travel path of the continuous sheet becomes closest to the arc track at the predetermined position,
the sheet-like member includes a lengthwise direction, a width direction and a thickness direction,
higher stretchability than that of a center portion in the width direction is imparted along the lengthwise direction to both end portions of the sheet-like member in the width direction, and
when the holding surface passes the predetermined position, the width direction of the sheet-like member held by the holding surface faces to a direction along the circulating trajectory.

According to such a manufacturing device of the composite body of the continuous sheet associated with the absorbent article, the sheet-like member whose end portions in the width direction each have stretchability can be extended across and fixed to the pair of continuous sheets while causing the lengthwise direction of the sheet-like member to face to an extending direction.

In the manufacturing device of the composite body of the continuous sheet associated with absorbent article,
it is preferable that the sheet-like member is transported toward a second predetermined position on the circulating trajectory in a form of a continuous body that continues in a transporting direction,
a plurality of the holding members is arranged in a direction along the circulating trajectory and circulates along the circulating trajectory, respectively,
when the holding surface passes the second predetermined position, the continuous body of the sheet-like member is sucked by and held on the holding surface with a suction force of the holding surface,
in magnitude of a suction force per unit area of the holding surface when passing the second predetermined position, magnitude of a portion that corresponds to a center portion of the continuous body in the width direction is smaller than that of a portion that corresponds to each of end portions of the continuous body in the width direction,
when the holding surface passes the second predetermined position, the center portion is held on the holding surface by the suction force after each of the end portions of the continuous body of the sheet-like member in the width direction is held, and
after being held on the holding surface, a portion of the continuous body, which is being held on the holding surface, is cut off from the continuous body by a cutter member to produce the sheet-like member.

According to such a manufacturing device of the composite body of the continuous sheet associated with the absorbent article, when the holding surface holds the continuous body of the sheet-like member at the above-mentioned second predetermined position, the holding surface holds the center portion after holding each of the end portions of the continuous body in the width direction. Thus, the holding surface can hold each of the end portions without being affected by the position to be held by the center portion of the holding surface. Thereby, each of the end portions can be correctly held at a position where each of the end portions should be held on the holding surface. Accordingly, each of the end portions can be fixed at the correct position in the pair of continuous sheets.

Further, since the sheet-like member is produced by being cut off from the continuous body after the continuous body of the sheet-like member is held on the holding surface, the cut-off process can be stably performed. Also, accuracy in producing the sheet-like member can be improved through improvement of accuracy of the cutting position.

In the manufacturing device of the composite body of the continuous sheet associated with absorbent article,
it is preferable that the sheet-like member is produced by being cut off from the continuous body by the cutter member in a state in which the lengthwise direction of the sheet-like member is along a continuous direction of the continuous body, and
while the produced sheet-like member is transported to the predetermined position by the holding member, the width direction of the sheet-like member is changed so as to face to a direction along the circulating trajectory due to a rotating operation of the holding member.

According to such a manufacturing device of the composite body of the continuous sheet associated with the absorbent article, the lengthwise direction of the sheet-like member can be directed to a direction along the above-mentioned rotational axis, that is, a direction intersecting with the travel direction of the pair of continuous sheets, and thus the sheet-like member can be promptly extended across the pair of continuous sheets.

In the manufacturing device of the composite body of the continuous sheet associated with absorbent article,
it is preferable that the holding surface of the holding member when passing the predetermined position is formed into a curved surface along the arc track except both end portions of the holding surface in a direction along the rotational axis, and each of the both end portions is provided with a cutout-shaped plane portion having such a chevron shape as to be cut out into a plane to increase the amount of cutout toward an end in a direction along the rotational axis, and
the cutout-shaped plane portion includes the part of the holding surface.

According to such a manufacturing device of the composite body of the continuous sheet associated with the absorbent article, the above-mentioned cutout-shaped plane portions are respectively provided in the both end portions of the holding surface in a direction along the rotational axis, and thus a part corresponding to the part described above can be readily provided.

Further,
a manufacturing method of a composite body of a continuous sheet associated with an absorbent article by extending a sheet-like member having four corners across a pair of continuous sheets that travels in parallel along a continuous direction and bonding the sheet-like member to the pair of continuous sheets, the method including:
causing a holding member that holds the sheet-like member on a holding surface to circulate along a circulating trajectory having a circular shape about a predetermined rotational axis, and
when the holding surface passes a predetermined position on the circulating trajectory in a state of facing outward in a radial direction of the circulating trajectory, bonding the sheet-like member to the pair of continuous sheets and delivering the sheet-like member to the pair of continuous sheets from the holding surface by pressing the pair of continuous sheets against the sheet-like member with an outer peripheral face of a roller member arranged according to the predetermined position,
the holding surface including portions that respectively correspond to the four corners of the sheet-like member, each of the portions being a curve surface-like portion along an arc track that is drawn by the each of the portions when passing the predetermined position,
a track that is drawn when at least a part that is arranged between two adjacent curve surface-like portions in a direction along the circulating trajectory on the holding surface passes the predetermined position being positioned more inwardly in the radial direction of the circulating trajectory than the arc track that is drawn when the two adjacent curve surface-like portions passes the predetermined position.

According to such a manufacturing method of the composite body of the continuous sheet associated with the absorbent article, each of the portions corresponding to the four corners of the sheet-like member on the holding surface is a curve surface-like portion along the above-mentioned arc track. Thus, the holding surface and the roller member can cooperate to certainly press the four corners of the sheet-like member against the pair of continuous sheets. Thereby, the four corners of the sheet-like member can be bonded to the pair of the continuous sheets with a high bonding strength, so that the sheet-like member can be firmly fixed in a state of being extended across the pair of continuous sheets.

Further, a track that is drawn by the above-mentioned part arranged between two curve surface-like portions is positioned more inwardly in the radial direction of the circulating trajectory than the arc track that is drawn by the two curve surface-like portions.

Thus, the pressing force per unit area that is imparted from the above-mentioned roller member to the above-mentioned part arranged between the two curve surface-like portions can be made smaller than the pressing force per unit area that is imparted from the roller member to the above-mentioned two curve surface-like portions. Accordingly, since the pressing force in this part can be reduced, flattened deformation of the outer peripheral face of the roller member can be mitigated and reduced at least in this part, thus suppressing creases of the sheet-like member.

### === Present Embodiment ===

A manufacturing device 30 of a composite body of a continuous sheet of the present embodiment is used, for example, in a manufacturing line of a disposable diaper 1.

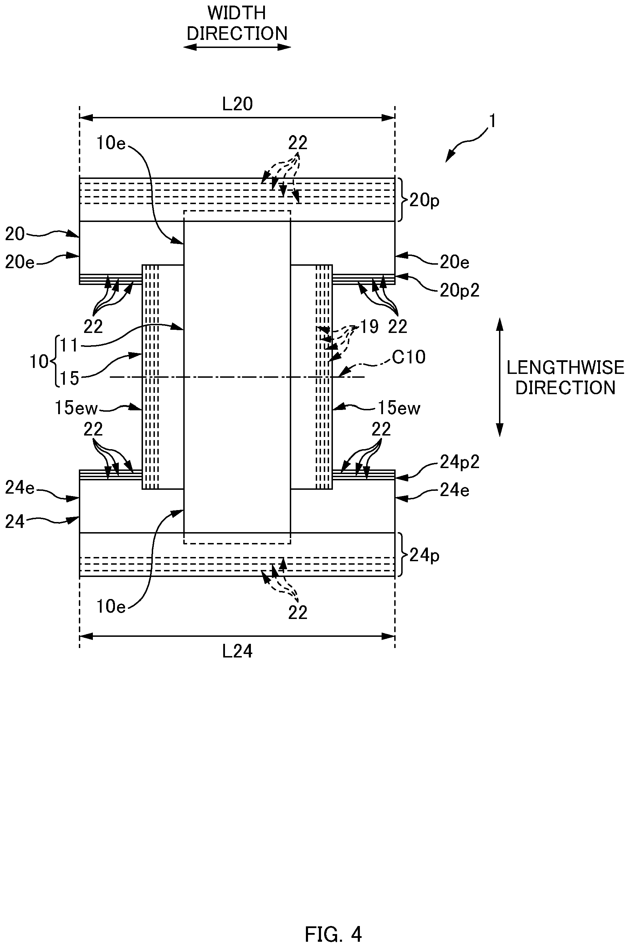

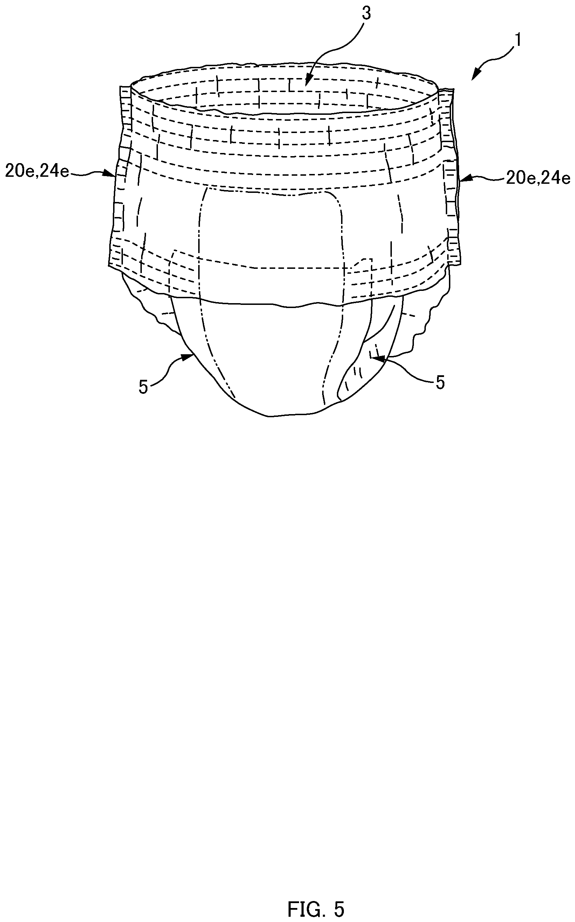

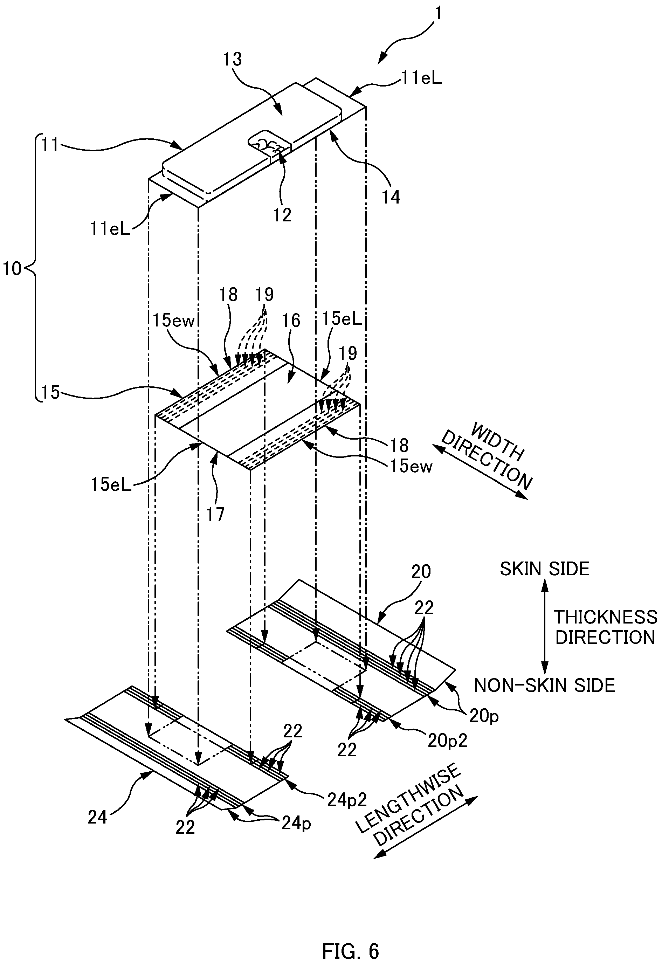

FIG. 4 to FIG. 6 are explanatory diagrams of the disposable diaper 1. FIG. 4 is a schematic plan view of the diaper 1 in a developed state, FIG. 5 is a schematic perspective view of the diaper 1 in a worn state, and FIG. 6 is a schematic exploded perspective view of the diaper 1 in a developed state.

This diaper 1 includes an abdominal side band member 20 that covers the abdominal side of the wearer, a back side band member 24 that covers the back side thereof, and a crotch member that is applied to the crotch thereof. In a developed state of FIG. 4, the abdominal side band member 20 and the back side band member 24 are in a state arranged side by side in parallel with a space therebetween, and both end portions 10e, 10e of the crotch member 10 in the lengthwise direction are extended across between these members 20, 24 and fixed thereto, so as to form a substantially H shape appearance when seen in a planar view.

From this state, the diaper 1 is folded in two at a substantially center portion C10 of the crotch member 10 in the lengthwise direction as a folding position. Further, in this two-folded state thereof, the band members 20, 24 opposite to each other are fastened at portions 20e, 24e which come into contact with the wearer's flanks. Thereby, these band members 20, 24 are connected in an annular manner. This results in the diaper 1 in a worn state in which a waist opening 3 and a pair of leg openings 5, 5 are formed as shown in FIG. 5.

As illustrated in FIG. 4 and FIG. 6, the abdominal side band member 20 and the back side band member 24 are both made of a soft sheet material such as non-woven fabric. In this example, each of the band members 20, 24 is formed by folding back the non-woven fabric and double layering parts 20p, 24p. Further, elastic members 22, 22 such as an elastic string are respectively interposed in an extended state in the double-layered parts 20p, 24p of the band members 20, 24, and are bonded and fixed to the double-layered parts 20p, 24p. Thereby, stretchability is imparted to these band members 20, 24. Note that, these double-layered parts 20p, 24p are parts that constitute a waist opening 3 of the diaper 1, and thus the waist opening 3 is stretchable due to this stretchability.

Further, in this example, in each of band members 20, 24, portions 20p2, 24p2 which constitute a part of the leg opening 5 are not double-layered. However, they may be double-layered. Further, in this example, elastic members 22, 22 such as an elastic string are also bonded and fixed in an extended state to the portions 20p2, 24p2 constituting a part of the leg opening 5, so that stretchability is also imparted to the portions 20p2, 24p2. Thereby, a part of the leg opening 5 is also stretchable.

As illustrated in FIG. 4 and FIG. 6, the crotch member 10 includes an absorbent main body 11 that absorbs excreted liquid such as urine of a wearer, and a flexible and thin exterior sheet member 15 provided closer to the non-skin side of the wearer than the absorbent main body 11.

The absorbent main body 11 includes an absorbent core 12 that is obtained by forming liquid absorbent material such as pulp fiber and SAP (superabsorbent polymer) in a predetermined shape, a liquid-permeable top sheet 13 which is provided to cover the absorbent core 12 from the skin side of the wearer, a liquid non-permeable back sheet 14 which is provided to cover the absorbent core 12 from the non-skin side of the wearer. The top sheet 13 is, for example, non-woven fabric. The back sheet 14 is, for example, a resin film such as polyethylene. Note that, the absorbent core 12 may be covered with a fluid-permeable sheet (not shown) such as tissue paper.

As illustrated in FIG. 6, the exterior sheet member 15 constitutes the exterior of the crotch member 10 and functions as a member forming a pair of leg openings 5, 5. Such an exterior sheet member 15 is a sheet member having a substantially rectangular shape and including a lengthwise direction, a width direction and a thickness direction. The lengthwise direction, the width direction and the thickness direction are directed in the lengthwise direction, width direction and thickness direction of the diaper 1 in a developed state of FIG. 4, respectively. Then, the exterior sheet member 15 projects on both sides in the width direction from the absorbent main body 11, and thus both end portions 15ew, 15ew of the exterior sheet member 15 in the width direction constitute a pair of leg openings 5, 5. Further, stretchability is imparted to the both end portions 15ew, 15ew in the lengthwise direction, and the stretchability of the portions 20p2, 24p2 of the foregoing band members 20, 24 is combined thereto, so that each of the leg openings 5, 5 is stretchable.

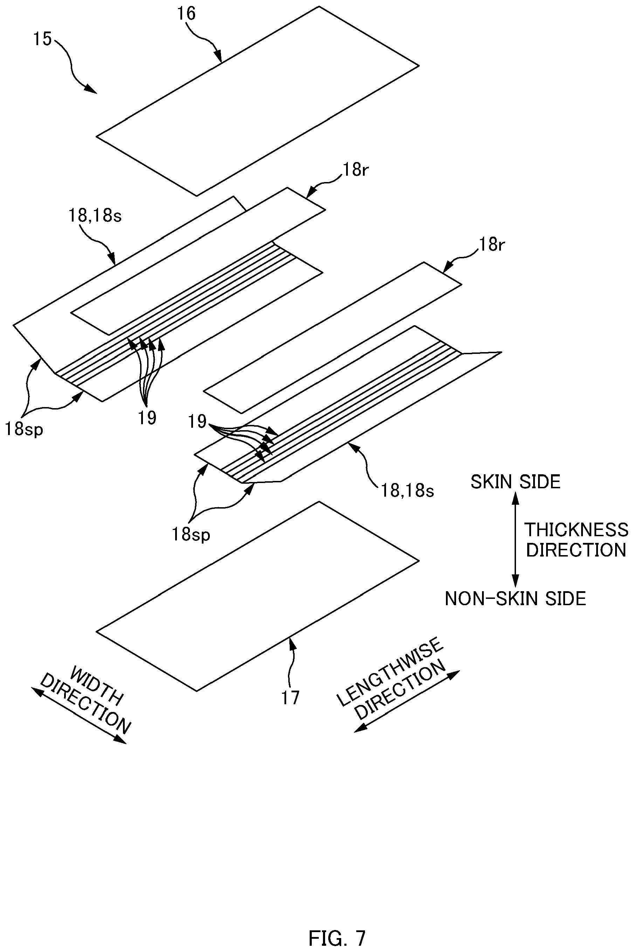

Note that, in this example, such an exterior sheet member 15 is not a single sheet but a composite sheet obtained by combining a plurality of sheets 16, 17, 18, 18 or the like. FIG. 7 is a schematic exploded perspective view of the exterior sheet member 15. As illustrated in FIG. 7, the exterior sheet member 15 includes a flexible skin side sheet 16 having a face on which the absorbent main body 11 is placed to be bonded and fixed, a flexible non-skin side sheet 17 that is overlaid to the skin side sheet 16 from the non-skin side to be bonded and fixed thereto, and a pair of stretchable sheets 18, 18 that is sandwiched between the skin side sheet 16 and the non-skin side sheet 17 at each end portion in the width direction, and is bonded and fixed to the both sheets 16, 17. The pair of stretchable sheets 18, 18 each projects from the skin side sheet 16 and the non-skin side sheet 17 in the width direction, and the projected portions constitute each of the end portions 15ew, 15ew of the exterior sheet member 15 in the width direction (FIG. 6).

A liquid non-permeable resin film can be exemplified as an example of the skin side sheet 16 in FIG. 7, and non-woven fabric can be exemplified as an example of the non-skin side sheet 17. Further, as the stretchable sheet 18, a sheet can be exemplified in which elastic members 19, 19 such as an elastic string are bonded and fixed to a flexible sheet 18s such as non-woven fabric in an extending state in the lengthwise direction, and in this example, such elastic members 19, 19 are interposed in a portion 18sp which has been double-layered by folding back the sheet 18s. Then, the stretchable sheet 18 is bonded and fixed to both the skin side sheet 16 and the non-skin side sheet 17 in a looser extending state than that of the elastic members 19, 19 at the time when the elastic members 19, 19 are bonded and fixed to the sheet 18s. Thus, the stretchable sheet 18 is fixed to both the sheets 16, 17 in a somewhat loose state in the lengthwise direction. In some cases, as illustrated in FIG. 7, a flexible reinforcing sheet 18r such as non-woven fabric may be interposed in the double-layered portion 18sp described above associated with the stretchable sheet 18, and be bonded and fixed thereto.

Incidentally, a process in which the crotch member 10 with such a configuration is extended across the pair of band members 20, 24 and is bonded thereto is performed in two steps as follows. First, as illustrated in FIG. 6, both end portions 15eL, 15eL in the lengthwise direction of the foregoing exterior sheet member 15 that constitutes a non-skin side half part of the crotch member 10 are extended across and fixed to a pair of band members 20, 24. Thereafter, the foregoing absorbent main body 11 that constitutes a skin-side half part of the crotch member 10 are overlapped on the exterior sheet member 15 from the skin side, and both end portions 11eL, 11eL of the absorbent main body 11 in the lengthwise direction are extended across and fixed to the pair of band members 20, 24 in the same manner as described above.

In some cases, a folding-back process described above for double-layering a pair of band members 20, 24 respectively may be performed after the absorbent main body 11 is extended across and fixed to a pair of band members 20, 24. Accordingly, the end portions 11eL, 11eL of the absorbent main body 11 are each interposed in the double-layered part 20p, 24p of the corresponding band members 20, 24, and this can reduce an uncomfortable feeling when the diaper 1 is worn. Thus, in this example, the above-mentioned configuration is employed (FIG. 4).

The diaper 1 as described above is manufactured in a manufacturing line. In the manufacturing line, an intermediate product 1m of the diaper 1 is transported along a predetermined transporting direction. During such transportation, various processes are applied to the intermediate product 1m, and each time each process is applied, the form of the intermediate product 1m sequentially changes, thereby finally completing the diaper 1 as shown in FIG. 5. The manufacturing device 30 of the present embodiment undertakes one of the processes.

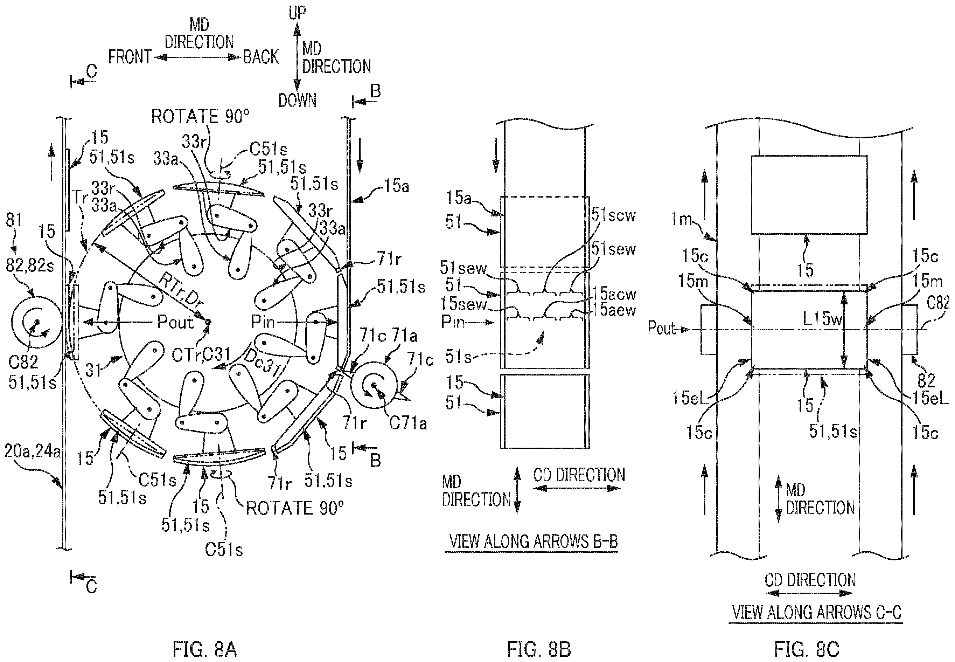

FIG. 8A is a schematic side view of the manufacturing device 30. Further, FIG. 8B is a view along arrows B-B in FIG. 8A, and FIG. 8C is a view along arrows C-C in FIG. 8A.

It should be noted that, hereinbelow, a width direction of the manufacturing line is also referred to as a "CD direction", and a direction that intersects this CD direction is also referred to as a "MD direction". In other words, the MD direction refers to any given direction in a plane that intersects with the CD direction. Then, the intermediate product 1m of the diaper 1 is transported using the MD direction as a transporting direction. Further, hereinbelow, in the two directions that intersect with each other in the MD direction, the vertical direction may be referred to as an "up-down direction", and the horizontal direction may be referred to as a "front-back direction" in some cases.

In this manufacturing device 30, basically, a process is performed in which the exterior sheet member 15 of the crotch member 10 is extended across and bonded to continuous bodies 20a, 24a of a pair of band members 20, 24. Thereby, a member 1m (corresponding to the composite body of the continuous sheet) of a substantially ladder shape as shown in FIG. 8C is manufactured as an intermediate product 1m of the diaper 1.

In the following, the above-mentioned process that is taken charge of by the manufacturing device 30 will be described in detail.

First, as illustrated in FIG. 8C, a pair of band members 20, 24 at the time of being supplied to the manufacturing device 30 is being transported along a continuous direction of the continuous bodies 20a, 24a in a form of the continuous bodies 20a, 24a along the MD direction, and in a state of being arranged in parallel with an interval between each other in the CD direction. Note that, in this example, each of the continuous bodies 20a, 24a of the pair of band members 20, 24 has not been folded-back yet, in other words, is transported in a state before being double-layered.

Further, the exterior sheet member 15 is also is transported along the continuous direction of the continuous body 15a in a form of a continuous body 15a that continues in the MD direction, as illustrated in FIG. 8B. In other words, the skin side sheet 16, the non-skin side sheet 17, a pair of stretchable sheets 18, 18 or the like that constitute the exterior sheet member 15 are each also be in a form of the continuous sheet that continues in the lengthwise direction of the exterior sheet member 15, and in a state where these continuous sheets are integral with one another by bonding or the like.

On the other hand, as illustrated in FIG. 8A, the manufacturing device 30 includes a rotating dram 31. The rotating dram 31 is driven and rotates about a rotational axis C31 along the CD direction. On an outer periphery of the rotating dram 31, a plurality of holding pads 51, 51 (corresponding to holding members) is provided lining in a rotational direction Dc31 of the rotating dram 31. The plurality of holding pads 51, 51 obtains a rotating force from the rotating operation of the rotating dram 31, and thus the pads 51 circulate along a predetermined circulating trajectory Tr having a perfect circle shape in one direction (in a clockwise direction in the example of FIG. 8A). A central axis CTr (corresponding to the "rotational axis" according to claims) of the circulating trajectory Tr having a perfect circle shape is also along the CD direction, and the central axis CTr is concentric with the rotational axis C31 of the rotating dram 31.

Each of the holding pads 51 includes a holding surface 51s that generates a suction force, and the holding surface 51s faces the outside in the radial direction Dr of the circulating trajectory Tr. Further, the continuous body 15a of the above-mentioned exterior sheet member 15 is transported toward a predetermined position Pin of the circulating trajectory Tr. Thus, when each of the holding pads 51, 51 passes the predetermined position Pin, each of the holding pads 51, 51 holds one side of the continuous body 15a of the exterior sheet member 15 by sucking it onto the holding surface 51s. That is, the predetermined position Pin is a receiving position Pin where each of the holding pads 51, 51 receives the continuous body 15a of the exterior sheet member 15.

Further, a cutter roll 71a is arranged as an example of a cutter member at a downstream position of the aforementioned receiving position Pin on the circulating trajectory Tr. The cutter roll 71a rotates around a rotational axis C71a that is along the CD direction, and cutter blades 71c, 71c, for example, whose tips are pointed, are provided on the outer peripheral face of the cutter roll 71a. Further, each of the holding pads 51 is provided with a receiving blade 71c that receives a cutter blade 71c, adjacent to the upstream end of the pad 51 in the circulating direction Dc31 at the receiving position Pin. Then, the cutter roll 71a rotates in conjunction with the rotating operation of the rotating dram 31 so as to allow the cutter blade 71c to be opposed to the receiving blade 71r when the receiving blade 71r passes by the position of the cutter roll 71a.

Thus, immediately after the holding pad 51 passes the arrangement position of the cutter roll 71a along the circulating trajectory Tr, a portion of the continuous body 15a of the exterior sheet member 15, which has been held by the holding pad 51, is cut off by being sandwiched between the receiving blade 71r adjacent to the upstream end of the holding pad 51 and the cutter blade 71c of the cutter roll 71a, thereby producing on the holding pad 51 a single sheet of exterior sheet member 15 whose lengthwise direction faces to the MD direction. Then, the holding pad 51 holds the single sheet of the exterior sheet member 15 on the holding surface 51s as it is, and moves downstream along the circulating trajectory Tr, thus moving the exterior sheet member 15 to a delivery position Pout (corresponding to a second predetermined position) on the circulating trajectory Tr.

Note that, in the process of moving to this delivery position Pout, the holding pad 51 rotates only 90 degrees about a planer center C51s of the holding surface 51s, and thus the exterior sheet member 15 which is on the holding surface 51 also rotates only 90 degrees about the planar center C51s. Consequently, the lengthwise direction of the exterior sheet member 15 is changed from the MD direction to the CD direction. Thereby, the exterior sheet member 15 becomes an attitude that can extend across the continuous bodies 20a, 24a of the pair of band members 20, 24.

On the other hand, the delivery position Pout is provided with a transport roller 82 (corresponding to a roller member) that guides the travel of the continuous bodies 20a, 24a of the pair of band members 20, 24. When the holding pad 51 passes the delivery position Pout, the transport roller 82 presses the continuous bodies 20a, 24a of the pair of band members 20, 24 against the exterior sheet member 15 being held by the holding pad 51. Thus, each of the portions 15eL, 15eL of the exterior sheet member 15 in the CD direction is bonded to the continuous bodies 20a, 24a of the pair of band members 20, 24a, respectively. Thereby, the exterior sheet member 15 is deliverd to the continuous bodies 20a, 24a of the pair of band members 20, 24 from the holding pad 51 to produce an intermediate product 1m having a substantially ladder shape as shown in FIG. 8C. That is, the intermediate product 1m having a substantially ladder shape is produced in which a plurality of exterior sheet members 15, 15 is bonded to the continuous bodies 20a, 24a of the pair of band members 20, 24 at a predetermined pitch in the MD direction.

The above is a processing range that is taken charge of by the manufacturing device 30. In this example, the continuous bodies 20a, 24a of the pair of band members 20, 24 correspond to "a pair of continuous sheets, and the exterior sheet member 15 corresponds to a "sheet-like member".

The process performed by this manufacturing device 30 was mainly explained as above. In the following, the detailed description of the configuration of this manufacturing device 30 will be given.

As illustrated in FIG. 8A, the rotating dram 31 is driven and rotates about the rotational axis C31 along the CD direction with a servomotor and the like as a driving source. On an outer periphery of the rotating dram 31, a plurality of arm members 33a, 33a is supported so as to be able to oscillate about a predetermined support point at each predetermined angle of the rotating dram 31 in the rotational direction Dc31. Then, the oscillating end of each arm member 33a is coupled to the corresponding holding pad 51 through an appropriate link member 33r. Further, each holding pad 51 is guided by an appropriate guide member (not shown) such as a guide rail, which has a perfect circle shape and is fixed to the grand side, so as to circulate along the aforementioned circulating trajectory Tr having a perfect circle shape. Thus, when the rotating dram 31 performs a rotating operation in one direction, a rotating force obtained by this rotating operation allows each of the holding pads 51, 51 to circulate along the circulating trajectory Tr in one direction (in a clockwise direction in the example of FIG. 8A) in conjunction with the rotating dram 31.

As illustrated in FIG. 8B and FIG. 8C, the holding surface 51s of the holding pad 51 includes the lengthwise direction and the width direction corresponding to a planar shape of the exterior sheet member 15 to be able to hold the exterior sheet member 15 in every corner. Then, as illustrated in FIG. 8B, at the receiving position Pin, the lengthwise direction of the holding surface 51s is made along the circulating trajectory Tr and the interval between the adjacent holding pads 51, 51 is narrowed. Then, the holding pad 51 passes the receiving position Pin in this state, sucks and holds the continuous body 15a of the exterior sheet member 15 on the holding surface 51s.

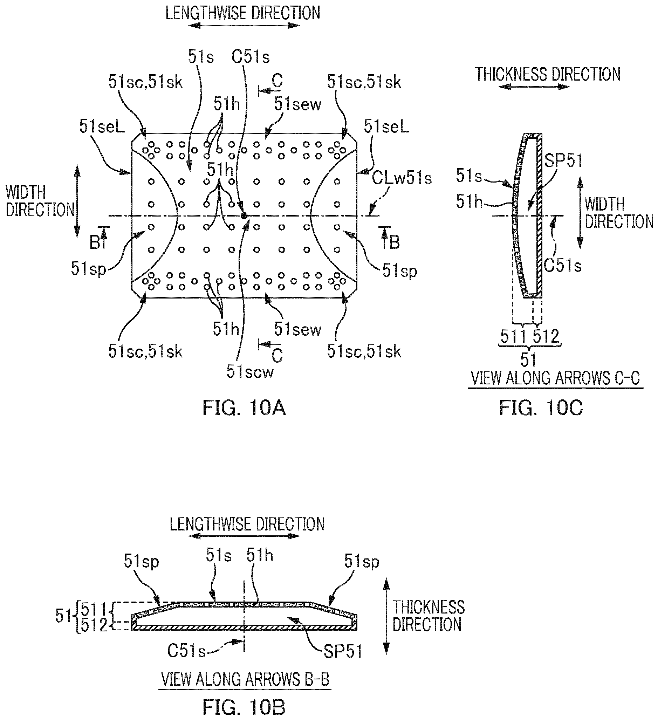

Here, a suction force of the holding surface 51s is generated by suction from a plurality of suction holes 51h, 51h that are provided on the holding surface 51s. In this example, according to the setting of a distribution density (in this example, it means the number of suction holes 51h, 51h per unit area, since respective diameters of the suction holes 51h, 51h are uniformly made the same with one another) of the suction holes 51h, 51h (see FIG. 10A because they are not shown in FIG. 8B), the suction force per unit area in both end portions 51sew, 51sew of the holding surface 51s in the width direction in FIG. 8B is allowed to be larger than the suction force per unit area in a center portion 51scw thereof.

Thus, at the receiving position Pin, after the holding surface 51s holds each of end portions 15aew, 15aew of the continuous body 15a of the exterior sheet member 15 in the width direction (CD direction), the holding surface 51s holds a center portion 15acw, as illustrated in FIG. 8B. Thereby, the holding surface 51s can hold each of the end portions 15aew, 15aew of the continuous body 15a of the exterior sheet member 15 in the width direction without being affected by the holding position where the continuous body 15a of the exterior sheet member 15 is to be held at the center portion 51scw of the holding surface 51s. Consequently, each of the end portions 15aew, 15aew of the continuous body 15a in the width direction can be correctly held at a position where each of the end portions 15aew, 15aew should be held on the holding surface 51s.

Note that, each of the end portions 15aew, 15aew corresponds to each of the end portions 15ew, 15ew of a single sheet of the exterior sheet member 15 in the width direction as shown in FIG. 6, and each of the end portions 15ew, 15ew is one of the important parts of the diaper 1, which constitute the leg openings 5, 5. Thus, when the suction force distribution as described above is set, attaching precision of such important parts can be improved.

Suction from the suction holes 51h, 51h can be achieved, for example, by partitioning and forming in the holding pad 51 a pressure chamber SP51 (see FIG. 10B and FIG. 10C) that is in communication with the suction holes 51h, 51h, and by connecting the above-mentioned pressure chamber SP51 to a negative pressure source (not shown) such as a blower or compressor through an appropriate unshown pipe member.

Meanwhile, as illustrated in FIG. 8A, the holding pad 51 holding the continuous body 15a of the exterior sheet member 15 passes by a position of the cutter roll 71a while substantially maintaining a state in which the interval between the adjacent holding pads 51 on the upstream side of the circulating trajectory Tr is narrowed. During this passage, the cutter blade 71c of the cutter roll 71a cooperates with the receiving blade 71r positioned between the holding pads 51, 51 to promptly cut off the continuous body 15a of the exterior sheet member 15, so that a part of the exterior sheet member 15 of the continuous body 15a, which has been held by the holding pad 51, is cut off from the continuous body 15a of the exterior sheet member 15. As a result, a single sheet of the exterior sheet member 15 whose lengthwise direction faces to the MD direction is produced on the holding pad 51.

Then, the holding pad 51 moves to a delivery position Pout while holding the exterior sheet member 15, rotates 90 degrees before reaching the delivery position Pout, and directs the lengthwise direction of the holding surface 51s to the CD direction.

However, when the holding pad 51 rotates 90 degrees in such a manner, there is a risk that the holding pads 51, 51 which are adjacent with each other in a direction along the circulating trajectory Tr interfere with each other. In other words, when one holding pad 51 rotates 90 degrees in a state in which the interval between the adjacent holding pads 51, 51 is narrowed, the holding pad 51 that has rotated 90 degrees may hit the adjacent holding pad 51. Further, before the holding pad 51 reaches the delivery position Pout, a pitch P51 between the adjacent holding pads 51, 51 also needs to be set in a size corresponding to lengths L20, L24 of the band members 20, 24 of the diaper 1 in FIG. 4.

For this reason, in this example, the pitch P51 between the adjacent holding pads 51, 51 is configured so as to be enlarged during the time before the holding pad 51 reaches the delivery position Pout after passing by the position of the cutter roll 71a. Further, it is also configured so that the holding pad 51 rotates 90 degrees again and the enlarged pitch P51 is narrowed during the time before the holding pad 51 reaches the receiving position Pin again after passing the delivery position Pout. Thus, when the holding pad 51 passes the receiving position Pin again, the lengthwise direction of the holding pad 51 is in a state of facing to the MD direction that is a direction along the circulating trajectory Tr, and the holding pad 51 returns to the state in which the interval between the holding pads 51, 51 is narrowed. Thus, receiving the continuous body 15a of the exterior sheet member 15 at the foregoing receiving position Pin can also be promptly achieved.

Each operation that enlarges or narrows the pitch P51 between such holding pads 51, 51 is achieved, for example, by a cam member. That is, an annular groove is formed, for example, as a front-face cam, in a not-shown cam member that is fixed to the ground side, and an engagement element such as a not-shown cam follower which engages with this annular groove is provided in each of the above-mentioned arm members 33a, 33a. Then, a cam curve that is a shape of the annular groove is set in a shape corresponding to patterns of the above-mentioned enlarging and narrowing operations. Thus, when the rotating dram 31 performs the rotating operation, the arm member 33a performs an oscillating operation corresponding to each rotational position in the rotational direction Dc31 of the rotating dram 31 by engaging the annular groove that is the front-face cam with the engagement element. Consequently, enlarging and narrowing the pitch P51 between the adjacent holding pads 51, 51 can be achieved.

Further, as described above with reference to FIG. 8A and FIG. 8C, the delivery position Pout is provided with a transport roller 82 to guide the travel of the continuous bodies 20a, 24a of the pair of band members 20, 24. The transport roller 82 rotates about a rotational axis C82 that is parallel to the CD direction, and the rotational axis C82 is immovably fixed in place. The continuous bodies 20a, 24a of the pair of band members 20, 24 come into contact to an outer peripheral face 82s of the transport roller 82, and the continuous bodies 20a, 24a of the pair of band members 20, 24 travel in the MD direction in a state of being arranged in the CD direction.

Such a transport roller 82 is a so-called flat roller. In other words, the transport roller 82 includes an outer peripheral face 82s having a perfect circle shape, whose center is the above-mentioned rotational axis C82. The radius of this perfect circle shape is maintained the same in a predetermined region in the CD direction, for example, at least over a region facing the holding surface 51s of the holding pad 51. Further, a surface layer portion including the outer peripheral face 82s of the transport roller 82 is constituted of an elastic material such as silicone rubber having a predetermined thickness, and thus the portion on which the pressing force acts from outward on the outer peripheral face 82s is allowed to be flatly deformed.

The transport roller 82 having such a configuration is positioned so as to cooperate with the holding surface 51s that passes the delivery position Pout and be able to press the continuous bodies 20a, 24a of the pair of band members 20, 24 against the exterior sheet member 15. In this example, the outer peripheral face 82s of the transport roller 82 becomes closest to the arc track drawn by the aftermentioned holding surface 51s' at the delivery position Pout, and is arranged so as to substantially contact the arc track. Thus, at the delivery position Pout, the travel direction of the continuous bodies 20a, 24a of the pair of band members 20, 24 is along the above-mentioned arc track, the travel path of the continuous bodies 20a, 24a becomes closest to the above-mentioned arc track at the delivery position Pout, and in this example, the travel path substantially contacts the above-mentioned arc track at the delivery position Pout.

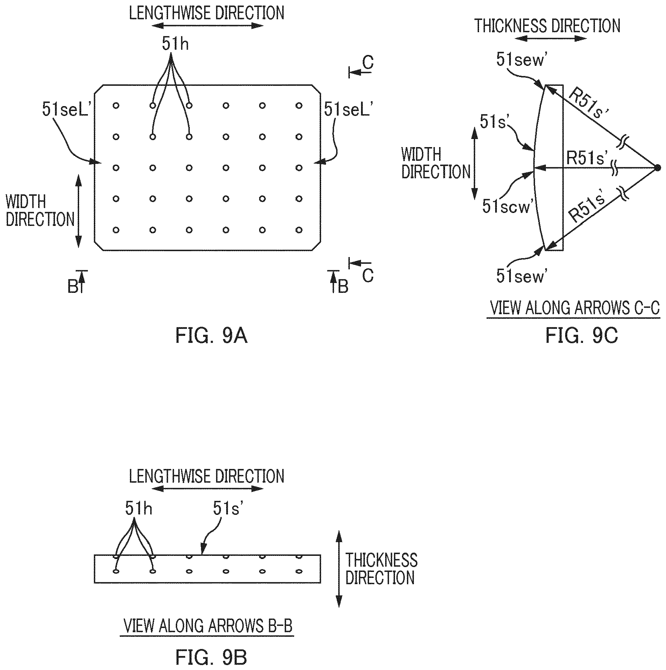

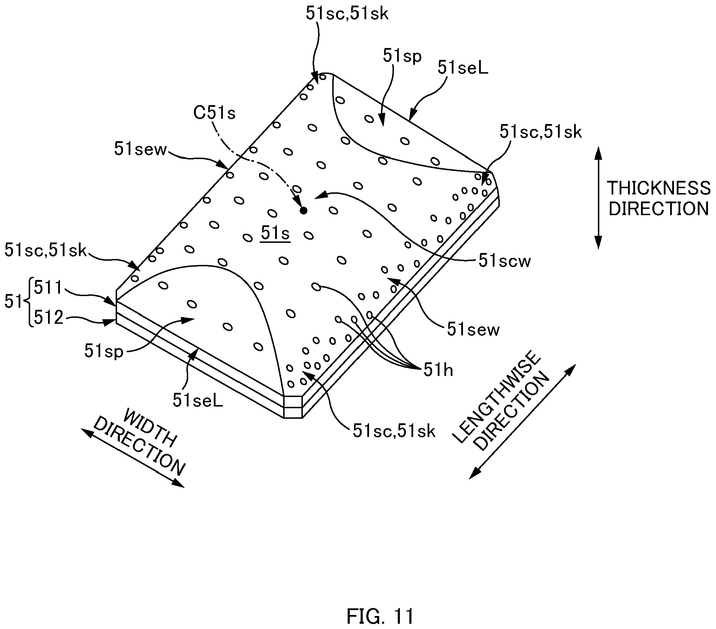

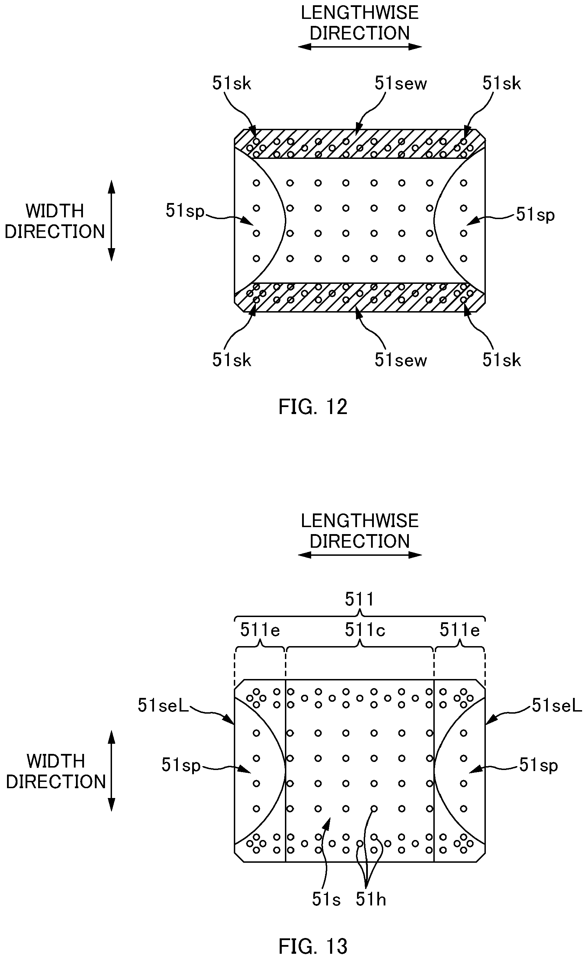

FIG. 9A to FIG. 10C are explanatory diagrams of the holding surface 51s of the holding pad 51. FIG. 9A to FIG. 9C are explanatory diagrams of the holding surface 51s' in the comparative example, and FIG. 10A to FIG. 10C are explanatory diagrams of the holding surface 51s of the present embodiment. Note that, the drawings both in the comparative example and the present embodiment show a front view (a view seen from outside of the circulating trajectory Tr in the radial direction Dr) of the holding surface 51s', 51s in FIG. A, show a view along arrows B-B of FIG. A in FIG. B, and show a view along arrows C-C of FIG. A in FIG. C. Further, FIG. 11 shows a schematic perspective view of the holding pad 51 of the present embodiment. Note that, in FIG. 9A to FIG. 11, the receiving blade 71r is not shown, and the same applies to FIG. 12 and FIG. 13 to be referred later.

As illustrated in FIG. 9A, the holding surface 51s' of the comparative example is a rectangular shape having a lengthwise direction and width direction when viewed from outside of the circulating trajectory Tr in the radial direction Dr. In the following, a direction orthogonal to both the lengthwise direction and the width direction of the holding surface 51s is also referred to as "thickness direction". Further, when the holding surface 51s is viewed from the width direction, as illustrated in FIG. 9B, the shape thereof is a flat shape along the lengthwise direction, and when viewed from the lengthwise direction, as illustrated in FIG. 9C, the shape thereof is a shape so as to gradually bulge in the thickness direction from each of the end portions 51sew' 51sew' in the width direction toward the center portion 51scw'. That is, the holding surface 51s' is an ark-like curved surface that curves in the width direction with a predetermined radius of curvature R51s'. Further, in other words, the sectional shape of the holding surface 51s' along the width direction is an ark shape of the above-mentioned radius of curvature R51s' as illustrated in FIG. 9C, and such an ark shape is maintained the same over the entire length in the lengthwise direction. Further, the above-mentioned radius of curvature R51s' is, for example, set to a value that is substantially equal to the shortest distance connecting the central axis CTr of the circulating trajectory Tr and the outer peripheral face 82s of the transport roller 82.

Thus, as shown in FIG. 8A, at the delivery position Pout, if the lengthwise direction of the holding surface 51s' faces to the CD direction and the width direction thereof faces to the MD direction that is a direction along the circulating trajectory Tr, the holding surface 51s' draws the same arc track over its entire surface when the holding surface 51s' passes the delivery position Pout.

Thereby, the transport roller 82 can press the holding surface 51s passing the delivery position Pout with a substantially uniform pressing force over the entire length of the holding surface 51 in the MD direction, and thus, as illustrated in FIG. 8C, the continuous bodies 20a, 24a of the pair of band members 20, 24 can be pressed against the exterior sheet member 15 with the substantially uniform pressing force over the entire length L15w of the exterior sheet member 15 in the MD direction. Consequently, both end portions 15eL, 15eL of the exterior sheet member 15 in the CD direction as shown in FIG. 8C is allowed to be bonded to the continuous bodies 20a, 24a of the pair of band members 20, 24 with a high bonding strength over the entire length L15w in the MD direction, respectively.

However, in the case of this comparative example, creases are caused on the exterior sheet member 15 due to the pressing by the above-mentioned transport roller 82. Also, there is a risk that the both end portions 15eL, 15eL of the exterior sheet member 15 in the CD direction may be bonded to the continuous bodies 20a, 24a of the pair of band members 20, 24 in a state of being creased.

In other words, as illustrated in FIG. 3, the pressing described above allows the outer peripheral face 82s of the transport roller 82 to be flatly deformed, and at that time, the length in the circumferential direction of this flatly deformed portion is shortened. Then, due to this shortening, a force F that compresses the exterior sheet member 15 in the MD direction may act on the exterior sheet member 15, and this force F readily causes small creases on the exterior sheet member 15. Note that, although such a force F may also act on the continuous bodies 20a, 24a of the pair of band members 20, 24, tension for travelling has acted on the continuous bodies 20a, 24a in the MD direction, and thus this tension in the MD direction may resist the above-mentioned force F. Accordingly, creases are not likely caused on the continuous bodies 20a, 24a, so that small creases are caused exclusively on the exterior sheet member 15 in which the above-mentioned tension does not act on in the MD direction. As a result, there is a possibility that the both end portions 15eL, 15eL of the exterior sheet member 15 in the CD direction may be bonded to the continuous bodies 20a, 24a of the pair of band members 20, 24 respectively, in such a state of being creased.

On the other hand, in view of bonding and fixing properties of the exterior sheet member 15 to the continuous bodies 20a, 24a of the pair of band members 20, 24, it is not necessary for the exterior sheet member 15 to be bonded to the continuous bodies 20a, 24a of the pair of band members 20, 24 with a high bonding strength over the entire length L15w (FIG. 8C) of the exterior sheet member 15 with respect to the MD direction that is a direction along the circulating trajectory Tr. That is, if at least four corners 15c, 15c, 15c, 15c of the exterior sheet member 15 are bonded with a high bonding strength as shown in FIG. 8C, it can be considered that the exterior sheet member 15 can be substantially firmly bonded and fixed to the continuous bodies 20a, 24a of the pair of band members 20, 24 even if a part 15m between adjacent two corners 15c, 15c in the direction along the circulating trajectory Tr is bonded with a lower bonding strength than that of the four corners 15c, 15c. Accordingly, it can be considered that there is no problem if the pressing force of the transport roller 82 against the part 15m between the two corners 15c, 15c is made weaker than that against the four corners 15c, 15c, and thus, it can be considered that this makes it possible to suppress creases of the exterior sheet member 15 due to the reduction of the flattened deformation of the transport roller 82.

Then, as illustrated in FIG. 10A to FIG. 11, the holding surface 51s of the holding pad 51 of the present embodiment has a shape such that both end portions 51seL', 51seL' on the holding surface 51s' of the holding pad 51' in the lengthwise direction of the comparative example in FIG. 9A are cut out into a plane.

More specifically, as illustrated in FIG. 10A to FIG. 11, both end portions 51seL, 51seL of the holding surface 51s in the lengthwise direction in the present embodiment are provided with portions 51sp, 51sp each having such a shape as to be cut out into a plane while the amount of cutout toward the ends in the lengthwise direction is increased. Accordingly, the cut-out portion 51sp is referred to as a chevron-shaped plane portion 51sp (hereinafter, also referred to as a cutout-shaped plane portion 51sp). Further, each of the cutout-shaped plane portions 51sp, 51sp described above is formed so that ark-like curved surface portions 51sk, 51sk, 51sk, 51sk remain respectively at the four corner portions 51sc, 51sc, 51sc, 51sc of the holding surface 51s.

Thus, when the holding surface 51s passes the delivery position Pout on the circulating trajectory Tr, each of arc-like curved surface portions 51sk, 51ks, 51sk, 51sk of the four corners on the holding surface 51s (corresponding to curve surface-like portions) draws the same arc track as described above. Therefore, the holding surface 51s cooperates with the transport roller 82 to allow the four corners 15c, 15c of the exterior sheet member 15 to be certainly pressed against the continuous bodies 20a, 24a of the pair of band members 20, 24. Thus, the four corners 15c, 15c of the exterior sheet member 15 can be bonded to the continuous bodies 20a, 24a of the pair of band members 20, 24 with a high bonding strength, thereby allowing the exterior sheet member 15 to be extended across and firmly fixed to the continuous bodies 20a, 24a of the pair of band members 20, 24.

On the other hand, each of cutout-shaped plane portions 51sp, 51sp of the both end portions 51seL, 51seL of the holding surface 51s in the lengthwise direction in FIG. 10A retracts more inwardly in the radial direction Dr of the circulating trajectory Tr than the arc-like curved surface portions 51sk, 51sk of the four corners. Thus, when the cutout-shaped plane portion 51sp passes the delivery position Pout, the cutout-shaped plane portion 51sp moves more inwardly in the radial direction Dr of the circulating trajectory Tr than the above-mentioned arc track drawn by the arc-like curved surface portions 51sk, 51sk of the four corners. That is, the track drawn by the plane portion 51sp when the cutout-shaped plane portion 51sp passes the delivery position Pout is positioned more inwardly in the radial direction Dr of the circulating trajectory Tr than the above-mentioned arc track drawn by the ark-like curved surface portions 51sk, 51sk of the four corners.

Thus, the pressing force per unit area to be imparted to the cutout-shaped plane portion 51sp from the transport roller 82 becomes smaller than the pressing force per unit area to be imparted to each of the ark-like curved surface portions 51sk, 51sk of the four corners from the transport roller 82, and this can mitigate and reduce the flattened deformation of the outer peripheral face 82s of the transport roller 82 in the cutout-shaped plane portion 51sp. Consequently, it is possible to suppress creases that may be caused in the both end portions 15eL, 15eL of the exterior sheet member 15 in the CD direction.

Further, as illustrated in FIG. 10A, a chevron shape that is a shape of the cutout-shaped plane portion 51sp is a line-symmetric shape with respect to the center line CLW51s in the width direction. Thus, a pressure distribution of the pressing force in the cutout-shaped plane portion 51sp can be uniformed in each portion of the both sides of the center line CLW51s. Thereby, an effect of suppressing creases and the bonding strength can be uniformed in each portion of the both sides of the center line CLW51s with each other.

The suction force per unit area of the arc-like curved surface portions 51sk, 51sk of the four corners on the holding surface 51s is made larger than the suction force per unit area of the portion other than the arc-like curved surface portions 51sk, 51sk of the four corners on the holding surface 51s. In this example, although hole diameters of the suction holes 51h, 51h are uniformed to be the same diameter over all of the suction holes 51h, 51h, the number of suction holes 51h, 51h per unit area of the holding surface 51s is greater in the arc-like curved surface portions 51sk, 51sk of the four corners, thus increasing the suction force per unit area in the arc-like curved surface portions 51sk, 51sk.

Thus, since the holding surface 51s can firmly hold the four corners 15c, 15c of the exterior sheet member 15 in the arc-like curved surface portions 51sk, 51sk of the four corners, the exterior sheet member 15 can be certainly restrained to be a state of being impossible to be displaced and deformed on the holding surface 15s. This effectively contributes to suppression of creases that may be caused on the exterior sheet member 15. In this example, as illustrated in FIG. 6, since stretchability in the lengthwise direction is imparted to both end portions 15ew, 15ew of the exterior sheet member 15 in the width direction, especially the shape of the both end portions 15ew, 15ew is not likely to be stable. However, as stated above, when the suction force in the arc-like curved surface portions 51sk, 51sk of the four corners is increased, the shape can be stabilized.

Further, it is preferable that a treatment for lowering adhesion is applied over the substantially entire face of the holding surface 51s in FIG. 10A. If such treatment has been applied, it is possible to prevent adhesion of adhesive to the holding surface 51s and problems accompanying the adhesion in advance. Details are as follows.

First, an adhesive such as a hot-melt adhesive is applied in advance to the exterior sheet member 15 or the continuous bodies 20a, 24a of the pair of band members 20, 24 for bonding with each other. Then, when the continuous bodies 20a, 24a of the pair of band members 20, 24 are pressed against and bonded to the exterior sheet member 15 of the holding surface 51s by the transport roller 82 at the delivery position Pout, there is a risk that the adhesive penetrates and oozes out in the thickness direction and then adheres to the holding surface 51s. Then, if the adhesive adheres thereto, it is difficult for the exterior sheet member 15 to separate from the holding surface 51s even after the exterior sheet member 15 is bonded to the continuous bodies 20a, 24a of the pair of band members 20, 24 at the above-mentioned delivery position Pout. As a result, the exterior sheet member 15 is not allowed to be smoothly delivered from the holding surface 51s to the continuous bodies 20a, 24a of the pair of band members 20, 24. However, with regard to this point, if the adhesion of the holding surface 51s is lowered as described above, the adhesive can be effectively prevented from adhering to the holding surface 51s, and thus the problems described above can also be prevented. Note that, as a specific example of the surface treatment that lowers the adhesion, a plasma coating can be exemplified. However, the treatment is not limited thereto.

Further, it is preferable that, as illustrated in FIG. 12, a friction coefficient of the arc-like curved surface portions 51sk, 51sk of the four corners on the holding surface 51s is larger than the friction coefficient of the cutout-shaped plane portions 51sp, 51sp on the holding surface 51s. In this example, a surface treatment for enhancing a friction coefficient is applied to both end portions 51sew, 51sew (parts shown by oblique lines in FIG. 12) of the holding surface 51s in the width direction, respectively, thereby enhancing a friction coefficient of the both end portions 51sew, 51sew over the substantially entire length in the lengthwise direction. However, on a part other than the both end portions 51sew, 51sew of the holding surface 51s in the width direction, the surface treatment described above is not applied, or a surface treatment for lowering a friction coefficient is applied, and thus the part other than the both end portions 51sew, 51sew is in a state of having a lower friction coefficient than that of the both end portions 51sew, 51sew. Here, the arc-like curved surface portions 51sk, 51sk of the four corncers are included in the former both end portions 51sew, 51sew in the width direction, and the cutout-shaped plane portions 51sp, 51sp are included in the latter part other than the both end portions 51sew, 51sew in the width direction.

Thus, the holding surface 51s can firmly hold the four corners 15c, 15c of the exterior sheet member 15 in the arc-like curved surface portions 51sk, 51sk of the four corners described above. Accordingly, the exterior sheet member 15 can be certainly restrained in a state of being impossible to be displaced and deformed on the holding surface 51s.

As a specific example of a surface treatment for enhancing a friction coefficient, a plasma coating having low adhesion and high grip performance can be exemplified. Further, as a specific example of a surface treatment for lowering the friction coefficient, a plasma coating having low adhesion and a sliding property can be exemplified. However, the treatment is not limited thereto. For example, the friction coefficient may be differentiated with different surface roughness.

Further, it is preferable that, as illustrated in FIG. 10B, FIG. 10C and FIG. 11, the holding pad 51 is configured so that it can be divided into two parts which are a holding surface side part 511 including the holding surface 51s, and a rotating dram side part 512 not including the holding surface 51s, and the former holding surface side part 511 is preferably fixed to the latter rotating dram side part 512 in a detachable fixing structure such as a bolt fastening. Then, if configured in this manner, when the holding surface 51s of the holding pad 51 deteriorates over time such as wear due to the pressing force of the transport roller 82, it is possible to readily repair the holding pad 51. In other words, if the holding surface side part 511 is removed from the rotating dram side part 512 of the holding pad 51 to be replaced with a new holding surface side part 511, it is possible to promptly repair the holding pad 51 of which the holding surface 51s has deteriorated over time.

Further, it is further preferable that, as illustrated in FIG. 13, the holding surface side part 511 is configured so that it can be divided into three parts in the lengthwise direction. That is, the holding surface side part 511 is preferably divided into three parts which are a pair of parts 511e, 511e being positioned at respective end portions 51seL, 51seL of the holding surface 51s in the lengthwise direction and each including the cutout-shaped plane portion 51sp, and a part 511c being positioned at the center portion in the lengthwise direction and not including the cutout-shaped plane portion 51sp. Then, if configured in this manner, it is possible to further reduce a unit of parts replacement which is caused by deterioration over time, thereby suppressing repair cost.

### === Other Embodiments ===

While the embodiments according to the present invention are described above, the present invention is not limited to the embodiments and can be altered as described below.

In the foregoing embodiment, the holding surface 51s of the holding pad 51 includes the chevron-like cutout-shaped plane portions 51sp, 51sp at both end portions 51seL, 51seL in the lengthwise direction, respectively. However, the present invention is not limited thereto. That is, in a case where the track that is drawn when at least a part that is arranged between the ark-like curved surface portions 51sk, 51sk of the two corners described above on the holding surface 51s passes the delivery position Pout is positioned more inwardly in the radial direction Dr of the circulating trajectory Tr than the arc track that is drawn when the ark-like curved surface portions 51sk, 51sk of the two corners which are adjacent with each other in the width direction on the holding surface 51s passes the delivery position Pout, reduction effect of the flattened deformation of the transport roller 82 described above can be accordingly provided, and creases can be suppressed. Thus, the part described above may not be the cutout-shaped plane portion 51sp. For example, a cutout-shaped curved surface part which is cut out into a curved surface may be provided in the both end portions 51seL, 51seL of the holding surface 51s in the lengthwise direction as the part describe above.

In the embodiment described above, the shape of the portion other than the cutout-shaped plane portion 51sp on the holding surface 51s is an ark-like curved surface as illustrated in FIG. 10A to FIG. 10C. However, the shape is not limited thereto. That is, as long as each of the shapes of the four corner portions 51sc, 51sc of the holding surface 51s is an ark-like curved surface, the shape of the portion other than the four corner portions 51sc, 51sc may not be an ark-like curved surface. For example, in addition to the above-mentioned cutout-shaped plane portion 51sp, 51sp of the both end portions 51seL, 51seL of the holding surface 51s in the lengthwise direction, the center portion 51scw of the holding surface 51s in the lengthwise direction may be dented inwardly in the radial direction Dr of the circulating trajectory Tr.

In the embodiment described above, the disposable diaper 1 is exemplified as an absorbent article. However, the present invention is not limited thereto as long as the absorbent article absorbs excreted liquid of humans and animals. For example, a disposable diaper for animals such as pets may also be applicable.

In the embodiment described above, the exterior sheet member 15 as one example of the sheet-like member is a composite sheet in which a plurality of sheets 16, 17, 18, 18 or the like is bonded. However, the exterior sheet is not limited thereto and may be formed by one sheet.

### [Reference Signs List]

1 disposable diaper, 1m intermediate product (composite body of a continuous sheet),
3 waist opening, 5 leg opening,
10 crotch member, 10e both end portions in lengthwise direction
11 absorbent main body, 11eL both end portions in lengthwise direction
12 absorbent core,
13 top sheet, 14 back sheet,
15 exterior sheet member (sheet-like member), 15a continuous body of exterior sheet member
15acw center portion, 15aew end portion
15c corner, 15eL both end portions in lengthwise direction,
15ew both end portions in width direction,
15m part,
16 skin side sheet, 17 non-skin side sheet, 18 stretchable sheet, 18r reinforcing sheet,
18s sheet, 18sp double-layered part,
19 elastic member
20 abdominal side band member, 20a continuous body (continuous sheet) of abdominal side band member, 20e portion,
20p double-layered part, 20p2 non double-layered part,
24 back side band member, 24a continuous body of back side band member (continuous sheet),
24p double-layered part, 24p2 non double-layered part,
30 manufacturing device,
31 rotating dram,
33a arm member, 33r link member,
51 holding pad (holding member), 51h suction hole,
51s holding surface, 51s' holding surface, 51sc four corner portions,
51scw center portion in the width direction, 51sew both end portions in the width direction,
51seL both end portions in the lengthwise direction,
51sk arc-like curved surface portion of four corners,
51sp cutout-shaped plane portion (plane portion),
71a cutter roll (cutter member), 71c cutter blade,
71r receiving blade,
81 delivery mechanism,
82 transport roller (roller member), 82s outer peripheral face,
511 holding surface side part, 511c part, 511e part,
512 rotating dram side part,
Tr circulating trajectory,
C10 substantially center portion,
C31 rotational axis, C51s planar center,
C71a rotational axis, C82 rotational axis,
CTr central axis (rotational axis),
CLW51s center line,
Pin receiving position (second predetermined position),
Pout delivery position (predetermined position),
SP51 pressure chamber,
R51s radius of curvature, RTr radius of circulating trajectory,

## Claims

1. A manufacturing device (30) of a composite body of a continuous sheet (1m) associated with an absorbent article by extending a sheet-like member (15) having four corners (15c) across a pair of continuous sheets (20a, 24a) that travels in parallel along a continuous direction and bonding the sheet-like member to the pair of continuous sheets, the device comprising:
a holding member (51) that circulates along a circulating trajectory (Tr) having a circular shape about a predetermined rotational axis (cTr), the holding member holding one side of the sheet-like member (15) on a holding surface (51s) that faces outward in a radial direction of the circulating trajectory; and
a roller member (82) arranged according to a predetermined position (Pout) on the circulating trajectory, an outer peripheral face (82s) of the roller member pressing the pair of continuous sheets (20a, 24a) toward the sheet-like member (15) when the holding surface (51s) passes the predetermined position (Pout), and thus causing the sheet-like member to be bonded to the pair of continuous sheets and delivered to the pair of continuous sheets from the holding surface,
the holding surface (51s) including portions (51sc) that respectively correspond to the four corners (15c) of the sheet-like member (15), each of the portions being a curve surface-like portion (51sk) along an arc track that is drawn by the each of the portions when passing the predetermined position (Pout),
a track that is drawn when at least a part that is arranged between two adjacent curve surface-like portions (51sk) in a direction along the circulating trajectory (Tr) on the holding surface (51s) passes the predetermined position (Pout) being positioned more inwardly in the radial direction (Dr) of the circulating trajectory than the arc track that is drawn when the two adjacent curve surface-like portions passes the predetermined position (Pout),
the holding surface (51s) sucking and holding the sheet-like member (15) with a suction force,
a suction force per unit area of the curve surface-like portion (51sk) on the holding surface being greater than that of a portion other than the curve surface-like portion on the holding surface.

2. A manufacturing device of a composite body of a continuous sheet associated with an absorbent article according to claim 1, wherein
a friction coefficient of the curve surface-like portion (51sk) is greater than that of the part that is arranged between the two curve surface-like portions on the holding surface.

3. A manufacturing device of a composite body of a continuous sheet associated with an absorbent article according to claim 1 or 2, wherein
a treatment for lowering adhesion is applied to the holding surface (51s).

4. A manufacturing device of a composite body of a continuous sheet associated with an absorbent article according to any one of claims 1 to 3, wherein
at the predetermined position (Pout), a travel direction of the continuous sheet (20a) is along the arc track, and a travel path of the continuous sheet becomes closest to the arc track at the predetermined position,
the sheet-like member (15) includes a lengthwise direction, a width direction and a thickness direction,
higher stretchability than that of a center portion in the width direction is imparted along the lengthwise direction to both end portions (15ew) of the sheet-like member in the width direction, and
when the holding surface (51s) passes the predetermined position, the width direction of the sheet-like member held by the holding surface faces to a direction along the circulating trajectory (Tr) .

5. A manufacturing device of a composite body of a continuous sheet associated with an absorbent article according to claim 4, wherein
the sheet-like member (15) is transported toward a second predetermined position (Pin) on the circulating trajectory (Tr) in a form of a continuous body (15a) that continues in a transporting direction,
a plurality of the holding members (51) is arranged in a direction along the circulating trajectory and circulates along the circulating trajectory, respectively,
when the holding surface (51s) passes the second predetermined position (Pin), the continuous body (15a) of the sheet-like member is sucked by and held on the holding surface with a suction force of the holding surface,
in magnitude of a suction force per unit area of the holding surface when passing the second predetermined position, magnitude of a portion that corresponds to a center portion (15acw) of the continuous body (15a) in the width direction is smaller than that of a portion that corresponds to each of end portions (15aew) of the continuous body in the width direction,
when the holding surface passes the second predetermined position, the center portion (15acw) is held on the holding surface (51s) by the suction force after each of the end portions (15aew) of the continuous body of the sheet-like member in the width direction is held, and
after being held on the holding surface, a portion of the continuous body (15a), which is being held on the holding surface, is cut off from the continuous body by a cutter member (71a) to produce the sheet-like member (15).

6. A manufacturing device of a composite body of a continuous sheet associated with an absorbent article according to claim 5, wherein
the sheet-like member (15) is produced by being cut off from the continuous body (15a) by the cutter member (71a) in a state in which the lengthwise direction of the sheet-like member is along a continuous direction of the continuous body, and
while the produced sheet-like member is transported to the predetermined position (Pout) by the holding member (51), the width direction of the sheet-like member is changed so as to face to a direction along the circulating trajectory (Tr) due to a rotating operation of the holding member.

7. A manufacturing device of a composite body of a continuous sheet associated with an absorbent article according to any one of claims 1 to 6, wherein
the holding surface (51s) of the holding member when passing the predetermined position (Pout) is formed into a curved surface (51sk) along the arc track except both end portions (51seL) of the holding surface in a direction along the rotational axis (CTr), and each of the both end portions (51seL) is provided with a cutout-shaped plane portion (51sp) having such a chevron shape as to be cut out into a plane to increase the amount of cutout toward an end in a direction along the rotational axis (cTr), and
the cutout-shaped plane portion (51sp) includes the part of the holding surface.

8. A manufacturing method of a composite body of a continuous sheet associated with an absorbent article by extending a sheet-like member (15) having four corners (15c) across a pair of continuous sheets (20a, 24a) that travels in parallel along a continuous direction and bonding the sheet-like member to the pair of continuous sheets, the method comprising:
causing a holding member (51) that holds the sheet-like member (15) on a holding surface (51s) to circulate along a circulating trajectory (Tr) having a circular shape about a predetermined rotational axis (cTr), and
when the holding surface (51s) passes a predetermined position (Pout) on the circulating trajectory (Tr) in a state of facing outward in a radial direction of the circulating trajectory (Tr), bonding the sheet-like member (15) to the pair of continuous sheets (20a, 24a) and delivering the sheet-like member (15) to the pair of continuous sheets (20a, 24a) from the holding surface (51s) by pressing the pair of continuous sheets against the sheet-like member with an outer peripheral face (82s) of a roller member (82) arranged according to the predetermined position (Pout),
the holding surface (51s) including portions that respectively correspond to the four corners (15c) of the sheet-like member (15), each of the portions being a curve surface-like portion (51sk) along an arc track that is drawn by the each of the portions when passing the predetermined position (Pout),
a track that is drawn when at least a part that is arranged between two adjacent curve surface-like portions (51sk) in a direction along the circulating trajectory (Tr) on the holding surface (51s) passes the predetermined position (Pout) being positioned more inwardly in the radial direction of the circulating trajectory (Tr) than the arc track that is drawn when the two adjacent curve surface-like portions passes the predetermined position,
the holding surface sucking and holding the sheet-like member with a suction force,
a suction force per unit area of the curve surface-like portion on the holding surface being greater than that of a portion other than the curve surface-like portion on the holding surface.

## Patentansprüche

1. Herstellungsvorrichtung (30) für einen zusammengesetzten Körper einer fortlaufenden Lage (1 m), die zu einem absorbierenden Artikel gehört, durch Ausbreiten eines lagenartigen Elements (15), das vier Ecken (15c) aufweist, über ein Paar von fortlaufenden Lagen (20a, 24a), die sich parallel entlang einer fortlaufenden Richtung bewegen, und Verbinden des lagenartigen Elements mit dem Paar von fortlaufenden Lagen, wobei die Vorrichtung Folgendes umfasst:
ein Halteelement (51), das entlang einer zirkulierenden Bahn (Tr), die eine kreisförmige Form um eine vorgegebene Drehachse (cTr) aufweist, zirkuliert, wobei das Halteelement eine Seite des lagenartigen Elements (15) auf einer Haltefläche (51s) hält, die in einer radialen Richtung der zirkulierenden Bahn nach außen zugewandt ist; und
ein Walzenelement (82), das entsprechend einer vorgegebenen Position (Pout) an der zirkulierenden Bahn angeordnet ist, wobei eine äußere Umfangsseite (82s) des Walzenelements das Paar von fortlaufenden Lagen (20a, 24a) in Richtung des lagenartigen Elements (15) drückt, wenn die Haltefläche (51s) die vorgegebene Position (Pout) passiert, und somit die Verbindung des lagenartigen Elements mit dem Paar von fortlaufenden Lagen und die Zuführung zu dem Paar von fortlaufenden Lagen von der Haltefläche bewirkt,
wobei die Haltefläche (51s) Abschnitte (51sc) einschließt, die jeweils den vier Ecken (15c) des lagenartigen Elements (15) entsprechen, wobei jeder der Abschnitte ein Kurvenflächen-artiger Abschnitt (51sk) entlang einer Bogenspur ist, die von jedem der Abschnitte gezogen wird, wenn die vorgegebene Position (Pout) passiert wird,
wobei eine Spur, die gezogen wird, wenn mindestens ein Teil, der zwischen zwei benachbarten Kurvenflächen-artigen Abschnitten (51sk) in einer Richtung entlang der zirkulierenden Bahn (Tr) auf der Haltefläche (51s) angeordnet ist, die vorgegebene Position (Pout) passiert, mehr nach innen in der radialen Richtung (Dr) der zirkulierenden Bahn positioniert ist als die Bogenspur, die gezogen wird, wenn die zwei benachbarten Kurvenflächen-artigen Abschnitte die vorgegebene Position (Pout) passieren,
wobei die Haltefläche (51s) das lagenartige Element (15) mit einer Saugkraft ansaugt und hält,
wobei eine Saugkraft pro Einheitsfläche des Kurvenflächen-artigen Abschnitts (51sk) auf der Haltefläche größer ist als die eines Abschnitts, der kein Kurvenflächen-artiger Abschnitt auf der Haltefläche ist.

2. Herstellungsvorrichtung eines zusammengesetzten Körpers einer fortlaufenden Lage, die zu einem absorbierenden Artikel gehört, nach Anspruch 1, wobei
ein Reibungskoeffizient des Kurvenflächen-artigen Abschnitts (51sk) größer ist als der des Teils, das zwischen den zwei Kurvenflächen-artigen Abschnitten auf der Haltefläche angeordnet ist.

3. Herstellungsvorrichtung eines zusammengesetzten Körpers einer fortlaufenden Lage, die zu einem absorbierenden Artikel gehört, nach Anspruch 1 oder 2, wobei
eine Behandlung für die Reduzierung der Haftung auf die Haltefläche (51s) angewendet wird.

4. Herstellungsvorrichtung eines zusammengesetzten Körpers einer fortlaufenden Lage, die zu einem absorbierenden Artikel gehört, nach einem der Ansprüche 1 bis 3, wobei
an der vorgegebenen Position (Pout) eine Bewegungsrichtung der fortlaufenden Lage (20a) entlang der Bogenspur verläuft und ein Bewegungsweg der kontinuierlichen Lage der Bogenspur an der vorgegebenen Position am nächsten kommt,
das lagenartige Element (15) eine Längsrichtung, eine Breitenrichtung und eine Dickenrichtung einschließt,
größere Dehnbarkeit als die eines mittleren Abschnitts in der Breitenrichtung entlang der Längsrichtung beiden Endabschnitten (15ew) des lagenartigen Elements in der Breitenrichtung verliehen wird und,
wenn die Haltefläche (51s) die vorgegebene Position passiert, die Breitenrichtung des lagenartigen Elements, das von der Haltefläche gehalten wird, einer Richtung entlang der zirkulierenden Bahn (Tr) zugewandt ist.

5. Herstellungsvorrichtung eines zusammengesetzten Körpers einer fortlaufenden Lage, die zu einem absorbierenden Artikel gehört, nach Anspruch 4, wobei
das lagenartige Element (15) in Richtung einer zweiten vorgegebenen Position (Pin) auf der zirkulierenden Bahn (Tr) in einer Form eines fortlaufenden Körpers (15a) befördert wird, der sich in einer Förderrichtung fortsetzt,
eine Vielzahl der Halteelemente (51) in einer Richtung entlang der zirkulierenden Bahn angeordnet ist und jeweils entlang der zirkulierenden Bahn zirkuliert,
wenn die Haltefläche (51s) die zweite vorgegebene Position (Pin) passiert, der fortlaufende Körper (15a) des lagenartigen Elements durch eine Saugkraft der Haltefläche angesaugt wird und auf der Haltefläche mit dieser gehalten wird,
in der Größenordnung einer Saugkraft pro Einheitsfläche der Haltefläche, wenn die zweite vorgegebene Position passiert wird, die Größenordnung eines Abschnitts, der einem mittleren Abschnitt (15acw) des fortlaufenden Körpers (15a) in der Breitenrichtung entspricht, kleiner ist als die eines Abschnitts, der jedem der Endabschnitte (15aew) des fortlaufenden Körpers in der Breitenrichtung entspricht,
wenn die Haltefläche die zweite vorgegebene Position passiert, der mittlere Abschnitt (15acw) auf der Haltefläche (51s) durch die Saukraft gehalten wird, nachdem jeder der Endabschnitte (15aew) des fortlaufenden Körpers des lagenartigen Elements in der Breitenrichtung gehalten wird, und
nach dem Halten auf der Haltefläche ein Abschnitt des fortlaufenden Körpers (15a), der auf der Haltefläche gehalten wird, von dem fortlaufenden Körper durch ein Schneideelement (71a) abgeschnitten wird, um das lagenartige Element (15) zu erzeugen.

6. Herstellungsvorrichtung eines zusammengesetzten Körpers einer fortlaufenden Lage, die zu einem absorbierenden Artikel gehört, nach Anspruch 5, wobei
das lagenartige Element (15) durch Abschneiden von dem fortlaufenden Körper (15a) durch das Schneideelement (71a) in einem Zustand erzeugt wird, in dem die Längsrichtung des lagenartigen Elements entlang einer fortlaufenden Richtung des fortlaufenden Körpers verläuft, und
während das erzeugte lagenartige Element zu der vorgegebenen Position (Pout) durch das Halteelement (51) befördert wird, die Breitenrichtung des lagenartigen Elements derart verändert wird, dass sie einer Richtung entlang der zirkulierenden Bahn (Tr) aufgrund eines Drehvorgangs des Halteelements zugewandt ist.

7. Herstellungsvorrichtung eines zusammengesetzten Körpers einer fortlaufenden Lage, die zu einem absorbierenden Artikel gehört, nach einem der Ansprüche 1 bis 6, wobei
die Haltefläche (51s) des Halteelements, wenn die vorgegebene Position (Pout) passiert wird, in eine Kurvenfläche (51sk) entlang der Bogenspur, mit Ausnahme der beiden Endabschnitte (51seL) der Haltefläche, in einer Richtung entlang der Drehachse (CTr) ausgebildet ist und jeder der beiden Endabschnitte (51seL) mit einem Ausschnittförmigen ebenen Abschnitt (51sp) bereitgestellt wird, der eine derartige Winkelform aufweist, um in eine Ebene geschnitten zu sein, um die Menge an Ausschnitt in Richtung eines Endes in einer Richtung entlang der Drehachse (cTr) zu erhöhen, und
der Ausschnitt-förmige ebene Abschnitt (51sp) das Teil der Haltefläche einschließt.

8. Herstellungsverfahren für einen zusammengesetzten Körper einer fortlaufenden Lage, die zu einem absorbierenden Artikel gehört, durch Ausbreiten eines lagenartigen Elements (15), das vier Ecken (15c) aufweist, über ein Paar von fortlaufenden Lagen (20a, 24a), die sich parallel entlang einer fortlaufenden Richtung bewegen, und Verbinden des lagenartigen Elements mit dem Paar von fortlaufenden Lagen, wobei das Verfahren Folgendes umfasst:
Bewirken, dass ein Halteelement (51), das das lagenartige Element (15) auf einer Haltefläche (51s) hält, entlang einer zirkulierenden Bahn (Tr), die eine kreisförmige Form um eine vorgegebene Drehachse (cTr) aufweist, zirkuliert, und,
wenn die Haltefläche (51s) eine vorgegebene Position (Pout) auf der zirkulierenden Bahn (Tr) in einem Zustand passiert, in dem sie in einer radialen Richtung der zirkulierenden Bahn (Tr) nach außen gewandt ist, Verbinden des lagenartigen Elements (15) mit dem Paar von fortlaufenden Lagen (20a, 24a) und Zuführen des lagenartigen Elements (15) zu dem Paar von fortlaufenden Lagen (20a, 24a) von der Haltefläche (51s) durch Drücken des Paars von fortlaufenden Lagen gegen das lagenartige Element mit einer äußeren Umfangsseite (82s) eines Walzenelements (82), das entsprechend der vorgegebenen Position (Pout) angeordnet ist,
wobei die Haltefläche (51s) Abschnitte einschließt, die jeweils den vier Ecken (15c) des lagenartigen Elements (15) entsprechen, wobei jeder der Abschnitte ein Kurvenflächen-artiger Abschnitt (51sk) entlang einer Bogenspur ist, die von jedem der Abschnitte gezogen wird, wenn die vorgegebene Position (Pout) passiert wird,
wobei eine Spur, die gezogen wird, wenn mindestens ein Teil, der zwischen zwei benachbarten Kurvenflächen-artigen Abschnitten (51sk) in einer Richtung entlang der zirkulierenden Bahn (Tr) auf der Haltefläche (51s) angeordnet ist, die vorgegebene Position (Pout) passiert, mehr nach innen in der radialen Richtung der zirkulierenden Bahn (Tr) positioniert ist als die Bogenspur, die gezogen wird, wenn die zwei benachbarten Kurvenflächen-artigen Abschnitte die vorgegebene Position passieren,
wobei die Haltefläche das lagenartige Element mit einer Saugkraft ansaugt und hält,
wobei eine Saugkraft pro Einheitsfläche des Kurvenflächen-artigen Abschnitts auf der Haltefläche größer ist als die eines Abschnitts, der kein Kurvenflächen-artiger Abschnitt auf der Haltefläche ist.

## Revendications

1. Dispositif de fabrication (30) d'un corps composite d'une feuille continue (1m) en association avec un article absorbant par l'extension d'un élément similaire à une feuille (15) ayant quatre coins (15c) en travers d'une paire de feuilles continues (20a, 24a) avançant en parallèle le long d'une direction continue et reliant l'élément similaire à une feuille à la paire de feuilles continues, le dispositif comportant :
un élément de retenue (51) qui circule le long d'une trajectoire de circulation (Tr) ayant une forme circulaire autour d'un axe de rotation prédéterminé (cTr), l'élément de retenue retenant un côté de l'élément similaire à une feuille (15) sur une surface de retenue (51s) qui est orientée vers l'extérieur dans une direction allant dans le sens radial de la trajectoire de circulation ; et
un élément formant rouleau (82) agencé en fonction d'une position prédéterminée (Pout) sur la trajectoire de circulation, une face périphérique extérieure (82s) de l'élément formant rouleau comprimant la paire de feuilles continues (20a, 24a) vers l'élément similaire à une feuille (15) quand la surface de retenue (51s) passe par la position prédéterminée (Pout), et amenant ainsi l'élément similaire à une feuille à être relié à la paire de feuilles continues et à être transporté jusqu'à la paire de feuilles continues depuis la surface de retenue,
la surface de retenue (51s) comprenant des parties (51sc) qui correspondent respectivement aux quatre coins (15c) de l'élément similaire à une feuille (15), chacune des parties étant une partie similaire à une surface courbe (51sk) le long d'un chemin en arc qui est tracé par lesdites chacune des parties lors du passage par la position prédéterminée (Pout),
un chemin qui est tracé quand au moins une partie qui est agencée entre deux parties similaires à une surface courbe adjacentes (51sk) dans une direction allant le long de la trajectoire de circulation (Tr) sur la surface de retenue (51s) passe par la position prédéterminée (Pout) qui est positionnée plus vers l'intérieur dans la direction allant dans le sens radial (Dr) de la trajectoire de circulation par rapport au chemin en arc qui est tracé quand les deux parties similaires à une surface courbe adjacentes passent par la position prédéterminée (Pout),
la surface de retenue (51s) aspirant et retenant l'élément similaire à une feuille (15) au moyen d'une force d'aspiration,
une force d'aspiration par unité de surface de la partie similaire à une surface courbe (51sk) sur la surface de retenue étant supérieure à celle d'une partie autre que la partie similaire à une surface courbe sur la surface de retenue.

2. Dispositif de fabrication d'un corps composite d'une feuille continue en association avec un article absorbant selon la revendication 1, dans lequel
un coefficient de friction de la partie similaire à une surface courbe (51sk) est supérieur à celui de la partie qui est agencée entre les deux parties similaires à une surface courbe sur la surface de retenue.

3. Dispositif de fabrication d'un corps composite d'une feuille continue en association avec un article absorbant selon la revendication 1 ou la revendication 2, dans lequel
un traitement servant à abaisser l'adhésion est appliqué sur la surface de retenue (51s).

4. Dispositif de fabrication d'un corps composite d'une feuille continue en association avec un article absorbant selon l'une quelconque des revendications 1 à 3, dans lequel
au niveau de la position prédéterminée (Pout), une direction d'avance de la feuille continue (20a) va le long du chemin en arc, et un chemin d'avance de la feuille continue vient se trouver au plus prêt du chemin en arc au niveau de la position prédéterminée,
l'élément similaire à une feuille (15) comprend une direction allant dans le sens de la longueur, une direction allant dans le sens de la largeur et une direction allant dans le sens de l'épaisseur,
une extensibilité supérieure à celle d'une partie centrale dans la direction allant dans le sens de la largeur est communiquée le long de la direction allant dans le sens de la longueur aux deux parties d'extrémité (15ew) de l'élément similaire à une feuille dans la direction allant dans le sens de la largeur, et
quand la surface de retenue (51s) passe par la position prédéterminée, la direction allant dans le sens de la largeur de l'élément similaire à une feuille retenu par la surface de retenue est orientée vers une direction allant le long de la trajectoire de circulation (Tr).

5. Dispositif de fabrication d'un corps composite d'une feuille continue en association avec un article absorbant selon la revendication 4, dans lequel
l'élément similaire à une feuille (15) est transporté vers une deuxième position prédéterminée (Pin) sur la trajectoire de circulation (Tr) sous une forme d'un corps continu (15a) qui continue dans une direction allant dans le sens du transport,
une pluralité d'éléments de retenue (51) est agencée dans une direction allant le long de la trajectoire de circulation et circule le long de la trajectoire de circulation, respectivement,
quand la surface de retenue (51s) passe par la deuxième position prédéterminée (Pin), le corps continu (15a) de l'élément similaire à une feuille est aspiré par et retenu sur la surface de retenue au moyen d'une force d'aspiration de la surface de retenue,
en termes d'amplitude d'une force d'aspiration par unité de surface de la surface de retenue lors du passage par la deuxième position prédéterminée, l'amplitude d'une partie qui correspond à une partie centrale (15acw) du corps continu (15a) dans la direction allant dans le sens de la largeur est inférieure à celle d'une partie qui correspondant à chacune des parties d'extrémité (15aew) du corps continu dans la direction allant dans le sens de la largeur,
quand la surface de retenue passe par la deuxième position prédéterminée, la partie centrale (15acw) est retenue sur la surface de retenue (51s) par la force d'aspiration après la retenue de chacune des parties d'extrémité (15aew) du corps continu de l'élément similaire à une feuille dans la direction allant dans le sens de la largeur, et
après avoir été retenue sur la surface de retenue, une partie du corps continu (15a), qui est retenue sur la surface de retenue, est découpée en provenance du corps continu par un élément de découpe (71a) pour produire l'élément similaire à une feuille (15).

6. Dispositif de fabrication d'un corps composite d'une feuille continue en association avec un article absorbant selon la revendication 5, dans lequel
l'élément similaire à une feuille (15) est produit en étant découpé en provenance du corps continu (15a) par l'élément de découpe (71a) dans un état dans lequel la direction allant dans le sens de la longueur de l'élément similaire à une feuille se trouve le long d'une direction continue du corps continu, et
alors que l'élément similaire à une feuille qui a été produit est transporté jusqu'à la position prédéterminée (Pout) par l'élément de retenue (51), la direction allant dans le sens de la largeur de l'élément similaire à une feuille est changée de manière à être orientée dans une direction allant le long de la trajectoire de circulation (Tr) en raison d'une opération de rotation de l'élément de retenue.

7. Dispositif de fabrication d'un corps composite d'une feuille continue en association avec un article absorbant selon l'une quelconque des revendications 1 à 6, dans lequel
la surface de retenue (51s) de l'élément de retenue lors du passage par la position prédéterminée (Pout) est formée en une surface courbe (51sk) le long du chemin en arc à l'exception des deux parties d'extrémité (51seL) de la surface de retenue dans une direction allant le long de l'axe de rotation (CTr), et chacune des deux parties d'extrémité (51seL) comporte une partie plane en forme de découpe (51sp) ayant une telle forme de chevrons de manière à être découpée en un plan pour augmenter la quantité de découpe vers une extrémité dans une direction allant le long de l'axe de rotation (cTr), et
la partie plane en forme de découpe (51sp) comprend la partie de la surface de retenue.

8. Procédé de fabrication d'un corps composite d'une feuille continue en association avec un article absorbant par l'extension d'un élément similaire à une feuille (15) ayant quatre coins (15c) en travers d'une paire de feuilles continues (20a, 24a) avançant en parallèle le long d'une direction continue et reliant l'élément similaire à une feuille à la paire de feuilles continues, le procédé comportant les étapes consistant à :
amener un élément de retenue (51) qui retient l'élément similaire à une feuille (15) sur une surface de retenue (51s) à circuler le long d'une trajectoire de circulation (Tr) ayant une forme circulaire autour d'un axe de rotation prédéterminé (cTr), et
quand la surface de retenue (51s) passe par une position prédéterminée (Pout) sur la trajectoire de circulation (Tr) dans un état orienté vers l'extérieur dans une direction allant dans le sens radial de la trajectoire de circulation (Tr), relier l'élément similaire à une feuille (15) à la paire de feuilles continues (20a, 24a) et envoyer l'élément similaire à une feuille (15) jusqu'à la paire de feuilles continues (20a, 24a) depuis la surface de retenue (51s) en comprimant la paire de feuilles continues contre l'élément similaire à une feuille avec une face périphérique extérieure (82s) d'un élément formant rouleau (82) agencé en fonction d'une position prédéterminée (Pout),
la surface de retenue (51s) comprenant des parties qui correspondent respectivement aux quatre coins (15c) de l'élément similaire à une feuille (15), chacune des parties étant une partie similaire à une surface courbe (51sk) le long d'un chemin en arc qui est tracé par lesdites chacune des parties lors du passage par la position prédéterminée (Pout),
un chemin qui est tracé quand au moins une partie qui est agencée entre deux parties similaires à une surface courbe adjacentes (51sk) dans une direction allant le long de la trajectoire de circulation (Tr) sur la surface de retenue (51s) passe par la position prédéterminée (Pout) qui est positionnée plus vers l'intérieur dans la direction allant dans le sens radial de la trajectoire de circulation (Tr) par rapport au chemin en arc qui est tracé quand les deux parties similaires à une surface courbe adjacentes passent par la position prédéterminée,
la surface de retenue aspirant et retenant l'élément similaire à une feuille au moyen d'une force d'aspiration,
une force d'aspiration par unité de surface de la partie similaire à une surface courbe sur la surface de retenue étant supérieure à celle d'une partie autre que la partie similaire à une surface courbe sur la surface de retenue.
